# EUROPEAN PATENT APPLICATION

(11) **EP 3 960 824 A1**
(43) Date of publication of application: **02.03.2022**
(21) Application number: 20796032.9
(22) Date of filing: 20.04.2020
(51) Int. Cl.: C09D 17/00, C08K 5/3462, C08L 101/00, C09D 201/00, C09D 11/037, C09D 11/322, C09D 7/41, C09D 7/65, C09B 67/20

(54) **COLORING COMPOSITION CONTAINING ISOINDOLINE COMPOUND AND USE OF SAME**

(30) Priority: 22.04.2019 JP 2019081301
(71) Applicant: Toyo Ink SC Holdings Co., Ltd., Tokyo 104-8377 (JP); TOYOCOLOR CO., LTD., Tokyo, 104-8381 (JP)
(72) Inventor: MATSUMOTO, Takeshi, Tokyo 104-8381 (JP); KONO, Takayoshi, Tokyo 104-8381 (JP); TAKIJIRI, Kouhei, Tokyo 104-8381 (JP); INAI, Teppei, Tokyo 104-8381 (JP); NISHIDA, Kazufumi, Tokyo 104-8381 (JP)
(74) Representative: SSM Sandmair
(86) International application number: PCT/JP2020/017103
(87) International publication number: WO 2020/218262

(57) **Abstract**

A coloring composition which contains an isoindoline compound represented by formula (1) (wherein A represents a group that is expressed by formula (2), formula (3) or formula (4)) and a dispersion medium.

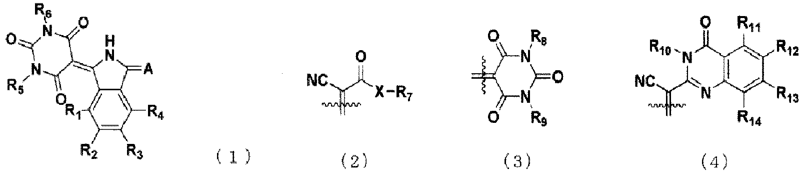

## Description

### [Technical Field]

The present disclosure relates to a coloring composition containing an isoindoline compound. In addition, the present disclosure relates to a colorant for plastics, a plastic molding material, a plastic molded product, a toner, a paint, a printing ink, and an inkjet ink, which include the coloring composition.

### [Background Art]

Pigments are typical colorants used in applications such as plastic products, toners, paints, and printing inks. Pigments are roughly classified into inorganic pigments and organic pigments, and organic pigments generally tend to be inferior in weather resistance and heat resistance as compared with inorganic pigments. However, organic pigments are used in various applications because they have better color clarity and coloring power than inorganic pigments. For example, azo pigments, quinophthalone pigments, isoindoline pigments, isoindolinone pigments, anthraquinone pigments, diketo-pyrrolo-pyrrole pigments, quinacridone pigments, and the like are known as organic pigments.

On the other hand, in recent years, in consideration of the environment, there has been an increasing demand for organic pigments and colorants which do not contain aromatic amines or heavy metals. Azo pigments are mainly used as organic pigments having a hue of yellow to crimson, but azo pigments may contain aromatic amines in their components due to the raw materials used or decomposition by light or heat. Therefore, in recent years, in consideration of environmental compatibility, isoindoline pigments containing no aromatic amines such as C. I. Pigment Yellow 185, C. I. Pigment Yellow 139, and C. I. Pigment Red 260 have been focused upon.

For example, Patent Literature 1 discloses a method of producing an asymmetric isoindoline pigment. Patent Literature 2 discloses an isoindoline pigment for coloring applications of plastics. Patent Literature 3 discloses a dispersion for an inkjet ink, which contains water, a dispersing agent, and an isoindoline pigment.

### [Citation List]

### [Patent Literature]

[Patent Literature 1] Japanese Patent Laid-Open No. H5-132630
[Patent Literature 2] Published Japanese Translation No. 2009-543917 of the PCT International Publication
[Patent Literature 3] Japanese Patent Laid-Open No. H10-140066

### [Summary of Invention]

### [Technical Problem]

However, in coloring compositions using an isoindoline pigment in the related art, the pigments themselves have low dispersibility and the weather resistance and heat resistance are not sufficiently satisfactory, and the pigments and compositions are limited in applications.

An objective of the present invention is to provide a coloring composition containing an isoindoline compound which has excellent dispersibility and excellent weather resistance and heat resistance and which can be applied to various applications.

### [Solution to Problem]

One embodiment of the present invention relates to a coloring composition including a pigment containing an isoindoline compound represented by the following Formula (1) and a dispersion medium:

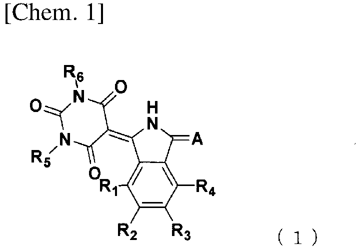

In the formula, R₁ to R₄ each independently represent a hydrogen atom, a halogen atom, a nitro group, an alkyl group, an alkoxy group, an aryl group, an aryloxy group, a thioalkyl group, or a thioaryl group. R₅ and R₆ each independently represent a hydrogen atom or an alkyl group, and at least one of R₅ and R₆ represents an alkyl group. A represent a group represented by the following Formula (2), the following Formula (3), or the following Formula (4).

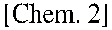

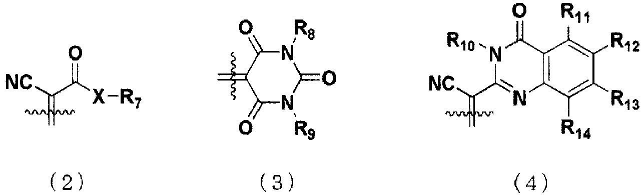

In the formulae, X represents -O- or -NH-, and R₇ represents an alkyl group or an aryl group.

R₅ to R₁₄ each independently represent a hydrogen atom, a halogen atom, an alkyl group, an alkoxy group, or an aryl group.

One embodiment relates to the coloring composition in which the dispersion medium contains a resin.

One embodiment relates to a colorant for plastics including the coloring composition according to the above embodiment.

One embodiment relates to a plastic molded product including the colorant for plastics according to the above embodiment.

One embodiment relates to a toner including the coloring composition according to the above embodiment.

One embodiment relates to the coloring composition in which the dispersion medium contains a resin and a solvent. The solvent preferably contains water.

One embodiment relates to a paint including the coloring composition according to the above embodiment.

One embodiment relates to a printing ink including the coloring composition according to the above embodiment.

One embodiment relates to an inkjet ink including the coloring composition according to the above embodiment.

### [Advantageous Effects of Invention]

According to the embodiment of the present invention, it is possible to provide a coloring composition containing an isoindoline compound which has excellent dispersibility, weather resistance and heat resistance and can be suitably used for various applications such as plastic coloring, paints, toners, and printing inks.

Priority is claimed on Japanese Patent Application No. 2019-081301, filed April 22, 2019, the content of which is incorporated herein by reference.

### [Brief Description of Drawings]

Fig. 1 is an X-ray diffraction spectrum of an isoindoline compound obtained in Synthesis Example 1-1 with Cu-Ka rays.
Fig. 2 is an X-ray diffraction spectrum of an isoindoline compound obtained in Synthesis Example 1-15 with Cu-Ka rays.

### [Description of Embodiments]

Hereinafter, embodiments of the present invention will be described in detail. However, the present invention is not limited to the following embodiments, and includes various embodiments. In this specification, unless otherwise specified, "(meth)acryloyl", "(meth)acrylic," "(meth)acrylic acid," "(meth)acrylate," and "(meth)acrylamide" indicate "acryloyl and/or methacryloyl," "acrylic and/or methacrylic," "acrylic acid and/or methacrylic acid," "acrylate and/or methacrylate," and "acrylamide and/or methacrylamide," respectively. In addition, "C. I." used in this specification is a color index (C. I.).

### <Coloring composition>

One embodiment relates a coloring composition containing a pigment containing Isoindoline Compound (1) and a dispersion medium.

### [Isoindoline Compound (1)]

In this specification, "Isoindoline Compound (1)" is a compound having an isoindoline framework represented by the following Formula (1). Isoindoline Compound (1) may include one or two or more compounds having an isoindoline framework represented by the following Formula (1). In addition, the crystal type of the compound having an isoindoline framework represented by the following Formula (1) is not limited, and any form may be used, and any crystal type compound can be suitably used in various embodiments to be described below. The content of Isoindoline Compound (1) is preferably 60 mass% or more, more preferably 70 mass% or more, and still more preferably 80 mass% or more based on the total mass of the pigment. In one embodiment, the content of Isoindoline Compound (1) in the pigment may be 100 mass%.

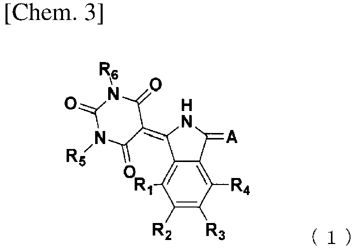

In Formula (1), R₁ to R₄ each independently represent a hydrogen atom, a halogen atom, a nitro group (-NO₂), an alkyl group, an alkoxy group, an aryl group, an aryloxy group, a thioalkyl group, or a thioaryl group.

R₅ and R₆ each independently represent a hydrogen atom or an alkyl group, and at least one of R₅ and R₆ is an alkyl group.

A represents a group represented by the following Formula (2), the following Formula (3), or the following Formula (4).

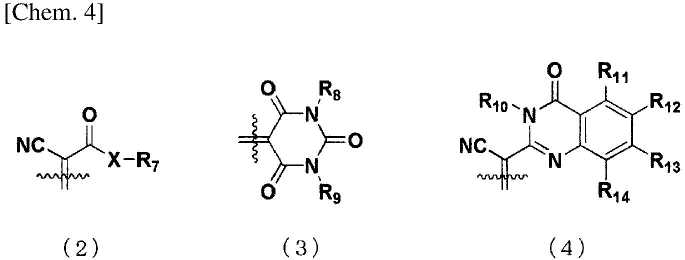

In Formula (2), X represents -O- or -NH-, and R₇ represents an alkyl group or an aryl group.

In Formula (3) and Formula (4), R₅ to R₁₄ each independently represent a hydrogen atom, a halogen atom, an alkyl group, an alkoxy group, or an aryl group.

In Formula (1), the halogen atom in R₁ to R₄ is not particularly limited, and may be, for example, a fluorine, chlorine, bromine, or iodine atom.

In Formula (1), the alkyl group (-R) in R₁ to R₄ is an atomic group obtained by removing one hydrogen atom from a hydrocarbon. The number of carbon atoms is preferably 1 to 20, more preferably 1 to 10, and still more preferably 1 to 6. The alkyl group may have any of a linear structure, a branched structure, a monocyclic structure, and a condensed polycyclic structure.

The alkyl group is not particularly limited, and examples thereof include a methyl group, ethyl group, propyl group, butyl group, pentyl group, hexyl group, heptyl group, octyl group, nonyl group, decyl group, dodecyl group, octadecyl group, isopropyl group, isobutyl group, isopentyl group, 2-ethylhexyl group, 2-hexyldodecyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group, norbornyl group, bornyl group, and 4-decylcyclohexyl group.

In the alkyl group, at least one hydrogen atom may be substituted with another substituent such as a fluorine atom. That is, the alkyl group may be a fluoroalkyl group or a perfluoroalkyl group. Specific examples of fluoroalkyl groups include a monofluoromethyl group, a difluoromethyl group, and a trifluoromethyl group.

The alkyl group may have a structure in which two or more alkyl groups (where one becomes an alkylene group) are bonded to each other via a linking group. Specific examples of linking groups include ester bonds (-COO-), ether bonds (-O-), and sulfide bonds (-S-). That is, in one embodiment, for example, the alkyl group may be a group represented by "-R'-O-R" (R' represents an atomic group obtained by removing one hydrogen atom from an alkyl group). Specific examples thereof include -C₂H₄-O-C₂H₅.

In one embodiment, the alkyl group is preferably an alkyl group having 1 to 6 carbon atoms, more preferably an alkyl group having 1 to 4 carbon atoms, and still more preferably an alkyl group having 1 or 2 carbon atoms.

In Formula (1), the alkoxy group in R₁ to R₄ is a group (-OR) in which an oxygen atom is bonded to the above alkyl group (-R).

In Formula (1), the aryl group (-Ar) in R₁ to R₄ is an atomic group obtained by removing one hydrogen atom from an aromatic hydrocarbon. The number of carbon atoms is preferably 6 to 30 and more preferably 6 to 20.

The aryl group is not particularly limited, and examples thereof include a phenyl group, biphenylyl group, terphenylyl group, quaternary phenylyl group, pentalenyl group, indenyl group, naphthyl group, binaphthyl group, ternaphthyl group, quaternary naphthyl group, azulenyl group, heptalenyl group, biphenylenyl group, indacenyl group, fluoranthenyl group, acephenanthrylene group, aceanthrylene group, phenalenyl group, fluorenyl group, anthryl group, anthracenyl group, teranthracenyl group, quaternary anthracenyl group, anthraquinone group, phenanthryl group, triphenylenyl group, pyrenyl group, chrysenyl group, naphthacenyl group, pleiadenyl group, picenyl group, perylene group, pentaphenyl group, pentacenyl group, tetraphenylene group, hexaphenyl group, hexadecenyl group, rubicenyl group, coronenyl group, trinaphthylenyl group, heptaphenyl group, heptacenyl group, phenanthleny group, and ovalenyl group.

In one embodiment, the aryl group is preferably a phenyl group.

In Formula (1), the aryloxy group in R₁ to R₄ is a group (-OAr) in which an oxygen atom is bonded to the above aryl group (-Ar). In one embodiment, the aryloxy group is preferably a phenoxy group.

In Formula (1), the thioalkyl group in R₁ to R₄ is a group (-SR) in which a sulfur atom is bonded to the above alkyl group. In one embodiment, the thioalkyl group is preferably a group in which a sulfur atom is bonded to an alkyl group having a linear structure. In addition, the thioaryl group is a group (-SAr) in which a sulfur atom is bonded to the above aryl group.

In one embodiment, the thioaryl group is preferably a thiophenyl group.

In one embodiment, in Formula (1), R₁ to R₄ are each independently preferably selected from the group consisting of a hydrogen atom, a nitro group, an alkyl group having 1 to 6 carbon atoms, a fluoroalkyl group having 1 to 6 carbon atoms, an alkoxy group (from an alkyl group having 1 to 6 carbon atoms), a phenyl group, a phenoxy group, a thioalkyl group (from an alkyl group having 1 to 6 carbon atoms), and a thiophenyl group.

In Formula (1), R₅, and R₆ each independently represent a hydrogen atom or an alkyl group. The number of carbon atoms of the alkyl group (-R) is preferably 1 to 20, more preferably 1 to 10, and still more preferably 1 to 6. The alkyl group may have any of a linear structure, a branched structure, a monocyclic structure, and a condensed polycyclic structure.

The alkyl group is not particularly limited, and examples thereof include a methyl group, ethyl group, propyl group, butyl group, pentyl group, hexyl group, heptyl group, octyl group, nonyl group, decyl group, dodecyl group, octadecyl group, isopropyl group, isobutyl group, isopentyl group, 2-ethylhexyl group, 2-hexyldodecyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group, norbornyl group, bornyl group, and 4-decylcyclohexyl group.

In the alkyl group, at least one hydrogen atom may be substituted with another substituent such as a fluorine atom. That is, the alkyl group may be a fluoroalkyl group or a perfluoroalkyl group. Specific examples of fluoroalkyl groups include a monofluoromethyl group, difluoromethyl group, and trifluoromethyl group.

In consideration of heat resistance, weather resistance, and dispersion stability, R₅ and R₆ are each more preferably an independently selected alkyl group than a hydrogen atom. That is, in one embodiment, at least one of R₅ and R₆ is an alkyl group, both are preferably an alkyl group, and more preferably, they are the same alkyl group. In one embodiment, the alkyl group is preferably an alkyl group having 1 to 6 carbon atoms, more preferably an alkyl group having 1 to 4 carbon atoms, and still more preferably an alkyl group having 1 or 2 carbon atoms.

In Formula (2), X represents -O- or -NH-, and is preferably -NH-.

In R₇, the alkyl group and the aryl group have the same meanings as in the above description. In consideration of heat resistance, weather resistance, and dispersion stability, the alkyl group is preferably an alkyl group having 1 to 4 carbon atoms and more preferably an alkyl group having 1 or 2 carbon atoms.

In Formula (3), R₅ and R₉ each independently represent a hydrogen atom or an alkyl group.

The number of carbon atoms of the alkyl group (-R) is preferably 1 to 20, more preferably 1 to 10, and still more preferably 1 to 6. The alkyl group may have any of a linear structure, a branched structure, a monocyclic structure, and a condensed polycyclic structure.

The alkyl group is not particularly limited, and examples thereof include a methyl group, ethyl group, propyl group, butyl group, pentyl group, hexyl group, heptyl group, octyl group, nonyl group, decyl group, dodecyl group, octadecyl group, trifluoromethyl group, isopropyl group, isobutyl group, isopentyl group, 2-ethylhexyl group, 2-hexyldodecyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group, norbornyl group, bornyl group, and 4-decylcyclohexyl group.

In consideration of heat resistance, weather resistance, and dispersion stability, R₅ and R₉ are more preferably an alkyl group than a hydrogen atom. In one embodiment, the alkyl group is preferably an alkyl group having 1 to 6 carbon atoms, more preferably an alkyl group having 1 to 4 carbon atoms, and still more preferably an alkyl group having 1 or 2 carbon atoms.

In Formula (4), the halogen atom, alkyl group, alkoxy group, and aryl group in R₁₀ to R₁₄ have the same meaning as in the above description of R₅ to R₆.

### [Method of producing Isoindoline Compound (1)]

Isoindoline Compound (1) used in the coloring composition of the present embodiment can be produced by a known synthesis method. For example, as shown in the following Scheme 1, it can be synthesized using a phthalonitrile represented by Formula (5) (hereinafter referred to as Compound (5)) or an isoindoline represented by Formula (6) (hereinafter referred to as Compound (6)) as a starting material.

Hereinafter, the synthesis method will be described with reference to specific examples of Isoindoline Compound (1). In the following description, the numbers stated in formulae will be described as compound numbers.

### (Compound in which A is Formula (2))

In one embodiment, Isoindoline Compound (1) can be produced according to the following Scheme 1.

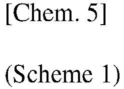

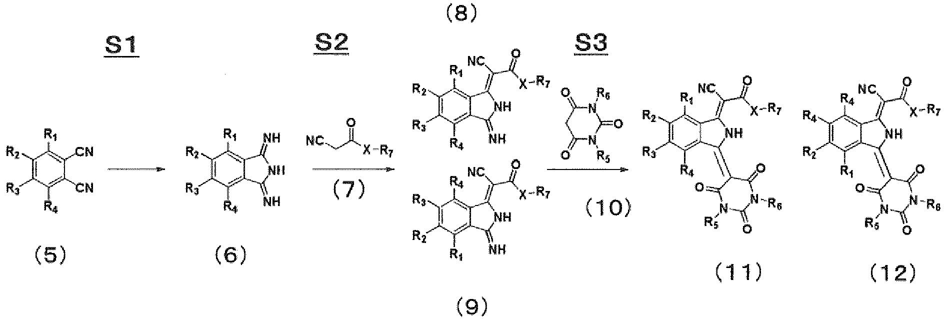

Scheme 1 may include a first step (S1) in which Compound (5) and a base are reacted in a solvent to obtain Compound (6); subsequently, a second step (S2) in which Compound (6) and Compound (7) are reacted in the presence of water; and subsequently a third step (S3) in which Compounds (8) and (9) and Compound (10) are reacted in the presence of acetic acid. The reaction temperature in each step in Scheme 1 is preferably 10 to 100°C.

When the relationship between substituents R₁ and R₄, and R₂ and R₃ of Compound (5) used as a starting material is asymmetric, the product produced by addition of Compound (7) is obtained as a mixture of isomers with different substituent positions, as represented as Compounds (8) and (9). Similarly, as represented as Compounds (11) and (12), the final product is also obtained as a mixture of isomers with different substituent positions.

In addition, when the relationship between the substituents R₅ and R₆ of Compound (10) is asymmetric, the final product is obtained as a mixture containing isomers. That is, when all the relationships between R₁ and R₄, between R₂ and R₃, and between R₅ and R₆ are asymmetric, theoretically, the final product is a mixture of four compounds (isomers).

On the other hand, when all the relationships between the substituents R₁ and R₄, and between R₂ and R₃ of Compound (5) used as a starting material and the relationship between the substituents R₅ and R₆ of Compound (10) are symmetric, the final product is a single compound.

Isoindoline Compound (1) constituting the coloring composition may be either a mixture containing isomers or a single compound.

Examples of solvents used in the first step (S1) include alcohols such as methanol, ethanol, isopropanol, butanol, and glycols, ethers such as glycol ether and tetrahydrofuran, and acyclic or cyclic amides such as formamide, dimethylformamide, dimethylacetamide, and N-methylpyrrolidone. Among these, an acyclic or cyclic amide is preferable, and tetrahydrofuran or formamide is more preferable.

The solvents exemplified above may be used alone or two or more thereof may be used in combination. The amount of the solvent used with respect to 100 parts by mass of Compound (5) is preferably 5 to 15 times and more preferably 5 to 10 times.

Examples of bases include alkali metal hydroxides, alkali metals such as lithium, sodium and potassium, alkali metal amides, and alkali metal hydrides; and alkali metals or alkaline earth metal alkoxides derived from a primary, secondary or tertiary aliphatic alcohols having an alkyl chain or alkylene chain having 1 to 10 carbon atoms. Among these, sodium hydroxide or potassium carbonate is preferable.

In addition, another synthesis method, for example, Isoindoline Compound (1) can be produced according to the following Scheme 2.

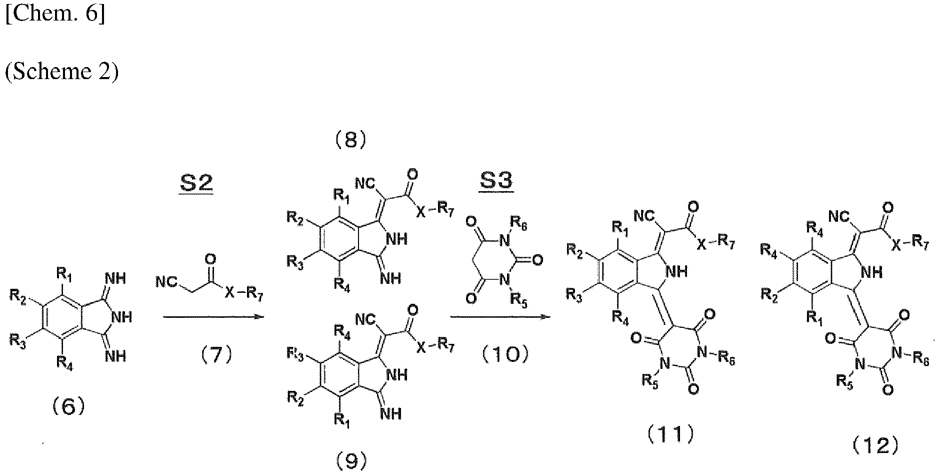

Scheme 2 may include a second step (S2) in which Compound (6) and Compound (7) are reacted in the presence of an aqueous ammonia solution; and subsequently a third step (S3) in which Compounds (8) and (9), and Compound (10) are reacted in the presence of acetic acid.

In the second step (S2) of Scheme 2, when a 28% aqueous ammonia solution is used, the amount of the aqueous ammonia solution used with respect to 100 parts by mass of Compound (6) is preferably 1 to 20 parts and more preferably 1 to 5 parts.

Although not particularly limited, Isoindoline Compound (1) used in the coloring composition of the present embodiment is preferably produced according to Scheme 2.

### (Compound in which A is Formula (3))

In one embodiment, Isoindoline Compound (1) can be produced according to the following Scheme 3.

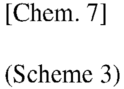

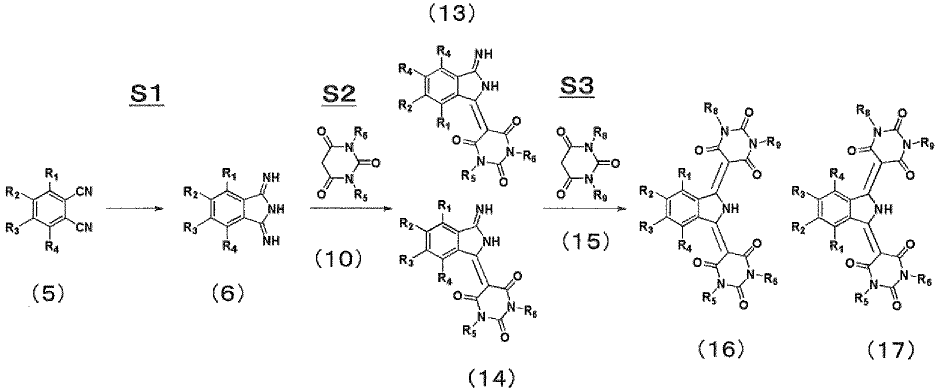

Scheme 3 may include a first step (S1) in which Compound (5) and a base are reacted in a solvent to obtain Compound (6); subsequently, a second step (S2) in which Compound (6) and Compound (10) are reacted in the presence of acetic acid; and subsequently a third step (S3) in which Compounds (13) and (14), and Compound (15) are reacted in the presence of acetic acid. The reaction temperature in each step in Scheme 3 is preferably 10 to 100°C.

When the relationships between the substituents R₁ and R₄, and between R₂ and R₃ of Compound (5) are asymmetric, the product produced by addition of Compound (10) is obtained as a mixture of isomers with different substituent positions, as represented as Compounds (13) and (14). Similarly, as represented as Compounds (16) and (17), the final product is also obtained as a mixture of isomers with different substituent positions.

In addition, when the relationship between the substituents R₅ and R₆ of Compound (10) is asymmetric, the product is obtained as a mixture containing isomers. In addition, when the relationship between the substituents R₅ and R₉ of Compound (15) is asymmetric, the product is obtained as a mixture containing isomers.

On the other hand, when all the relationships between the substituents R₁ and R₄, and between R₂ and R₃ of Compound (5), between the substituents R₅ and R₆ of Compound (10), and between the substituents R₅ and R₉ of Compound (15) are symmetric, the final product is a single compound.

Isoindoline Compound (1) constituting the coloring composition may be either a mixture containing isomers or a single compound.

Examples of solvents used in the first step (S1) include alcohols such as methanol, ethanol, isopropanol, butanol, and glycols; ethers such as glycol ether and tetrahydrofuran; and acyclic or cyclic amides such as formamide, dimethylformamide, dimethylacetamide, and N-methylpyrrolidone. Among these, an acyclic or cyclic amide is preferable, and tetrahydrofuran or formamide is more preferable.

The solvents may be used alone or two or more thereof may be used in combination. The amount of the solvent used with respect to 100 parts by mass of Compound (5) is preferably 5 to 15 times and more preferably 5 to 10 times.

Examples of bases include alkali metal hydroxides, alkali metals such as lithium, sodium and potassium, alkali metal amides, and alkali metal hydrides; and alkali metals or alkaline earth metal alkoxides derived from a primary, secondary or tertiary aliphatic alcohols having an alkyl chain or alkylene chain having 1 to 10 carbon atoms. Among these, sodium hydroxide or potassium carbonate is preferable.

In addition, another synthesis method, Isoindoline Compound (1) can be produced according to the following Scheme 4.

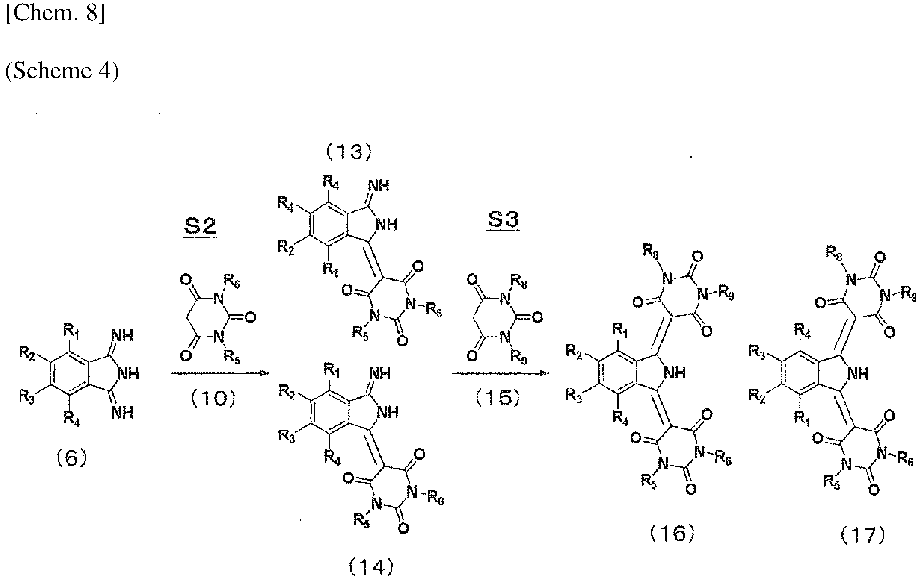

Scheme 4 may include a second step (S2) in which Compound (6) and Compound (10) are reacted in the presence of acetic acid; and subsequently, a third step (S3) in which Compounds (13) and (14), and Compound (15) are reacted in the presence of acetic acid.

### (Compound in which A is Formula (4))

In one embodiment, Isoindoline Compound (1) can be produced according to the following Scheme 5.

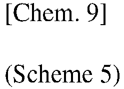

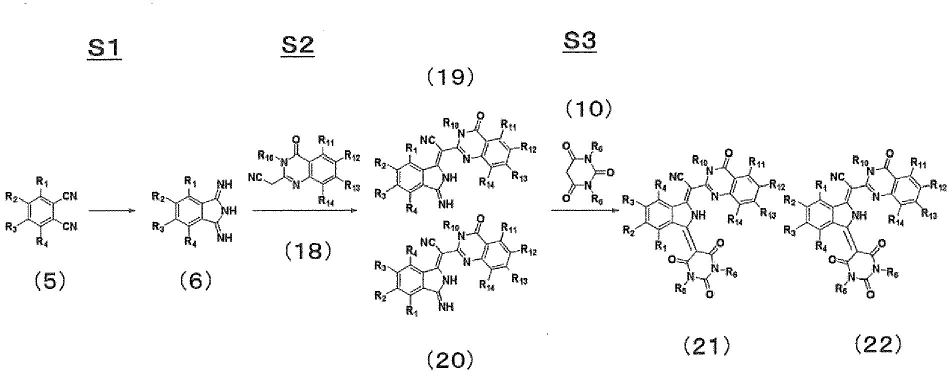

Scheme 5 may include a first step (S1) in which Compound (5) and a base are reacted in a solvent to obtain Compound (6); subsequently a second step (S2) in which Compound (6) and Compound (18) are reacted in the presence of water; and subsequently a third step (S3) in which Compounds (19) and (20), and Compound (10) are reacted in the presence of acetic acid. The reaction temperature in each step in Scheme 5 is preferably 10 to 100°C.

When the relationship between substituents R₁ and R₄, and R₂ and R₃ of Compound (5) used as a starting material is asymmetric, the product produced by addition of Compound (18) is obtained as a mixture of isomers with different substituent positions, as represented as Compounds (19) and (20). Similarly, as represented as Compounds (21) and (22), the final product is also obtained as a mixture of isomers with different substituent positions

In addition, when the relationship between the substituents R₅ and R₆ of Compound (10) is asymmetric, the final product is obtained as a mixture containing isomers.

On the other hand, when the relationships between the substituents R₁ and R₄, and between R₂ and R₃ of Compound (5) are symmetric, and the relationship between the substituents R₅ and R₆ of Compound (10) are symmetric, the final product is a single compound.

Isoindoline Compound (1) constituting the coloring composition may be either a mixture containing isomers or a single compound.

Examples of solvents used in the first step (S1) include alcohols such as methanol, ethanol, isopropanol, butanol, and glycols; ethers such as glycol ether and tetrahydrofuran; and acyclic or cyclic amides such as formamide, dimethylformamide, dimethylacetamide, and N-methylpyrrolidone. Among these, an acyclic or cyclic amide is preferable, and tetrahydrofuran or formamide is more preferable.

The solvents may be used alone or two or more thereof may be used in combination. The amount of the solvent used with respect to 100 parts by mass of Compound (5) is preferably 5 to 15 times and more preferably 5 to 10 times.

Examples of bases include alkali metal hydroxides, alkali metals such as lithium, sodium and potassium, alkali metal amides, and alkali metal hydrides; and alkali metals or alkaline earth metal alkoxides derived from a primary, secondary or tertiary aliphatic alcohols having an alkyl chain or alkylene chain having 1 to 10 carbon atoms. Among these, sodium hydroxide or potassium carbonate is preferable.

In addition, another synthesis method, Isoindoline Compound (1) can be produced according to the following Scheme 6.

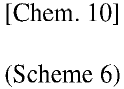

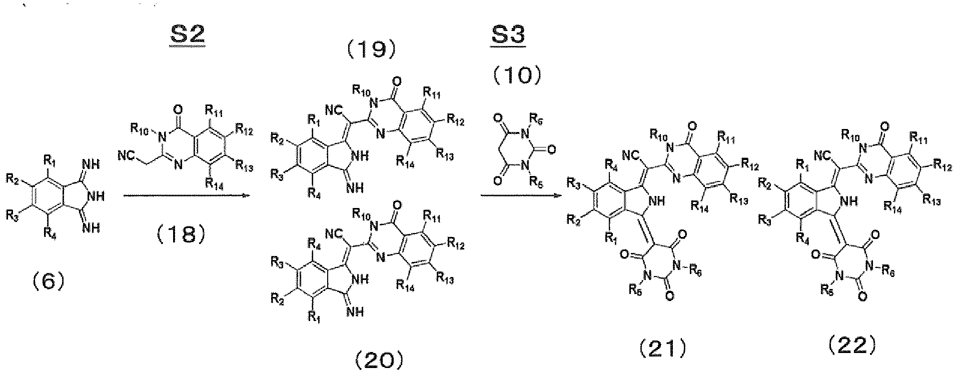

Scheme 6 includes a second step (S2) in which Compound (6) and Compound (18) are reacted in the presence of an aqueous ammonia solution; and subsequently a third step (S3) in which Compounds (19) and (20), and Compound (10) are reacted in the presence of acetic acid.

In the second step (S2) of Scheme 6, when a 28% aqueous ammonia solution is used, the amount of the aqueous ammonia solution used with respect to 100 parts by mass of Compound (6) is preferably 1 to 20 times and more preferably 1 to 5 times.

In the above Isoindoline Compound (1) used in the coloring composition according to the embodiment, at least one of R₅ and R₆ in the above Formula (1) is an alkyl group. When at least one alkyl group is introduced at a predetermined position in the isoindoline compound, compared to conventional isoindoline pigments that do not have an alkyl group at a predetermined position, the dispersibility, weather resistance, and heat resistance can be significantly improved. Therefore, the coloring composition can be suitably used in various applications such as aqueous paints, printing inks, and inkjet inks, for which it has been difficult to apply an isoindoline pigment in the related art.

Although not particularly limited, preferable forms of the above Isoindoline Compound (1) used in the coloring composition are as follows. Here, when there are isomers, compounds listed in tables may be mixtures containing these isomers. In addition, the C6H13 group and the Bu (butyl) group are both linear.

Specific examples of Compounds (11) and (12) shown in Schemes 1 and 2 are as follows.

**[Table 1]**

| R₁, R₄ | | R₂, R₃ | | R₅, R₆ | | R₇ | X |
|---|---|---|---|---|---|---|---|
| H | H | H | H | Me | Me | Me | NH |
| H | H | H | H | Me | Me | Cyclohexyl | NH |
| H | H | H | H | Et | Et | Et | NH |
| H | H | H | H | Me | Et | Me | NH |
| H | H | t-Bu | H | Me | Et | Me | NH |
| H | H | OPh | OPh | Me | Me | Me | NH |
| F | F | F | F | Me | Me | C₆H₁₃ | NH |
| H | H | Cl | H | Bu | Bu | Me | NH |
| H | H | H | H | CF₃ | CF₃ | Me | NH |
| H | H | NO₂ | H | Me | Me | Me | NH |
| SPh | SPh | H | H | Me | H | Et | NH |
| OMe | OMe | Br | Br | Me | Me | Me | NH |
| NO₂ | H | H | H | Me | Me | Me | NH |
| H | H | Me | Me | Me | Bu | Me | NH |
| Cl | Cl | Cl | Cl | Cyclohexyl | Cyclohexyl | Me | NH |
| Bu | Bu | H | H | Me | H | Et | NH |
| H | H | H | H | C₆H₁₃ | C₆H₁₃ | Me | NH |
| OMe | OMe | H | H | Me | Me | Me | NH |
| OMe | H | H | H | Me | H | Bu | NH |
| H | H | H | H | C₂H₄OC₂H₅ | C₂H₄OC₂H₅ | Me | NH |
| F | H | H | H | Bu | H | Me | NH |
| H | H | SMe | H | Me | H | Me | NH |
| OPh | H | H | H | Me | Me | Me | NH |
| Br | Br | Br | Br | Cyclobutyl | Cyclobutyl | Me | NH |
| H | H | CF₃ | CF₃ | Et | H | Bu | NH |
| H | H | H | H | Me | Me | Me | O |
| H | H | H | H | Et | H | Et | O |
| F | F | F | F | Me | Me | Me | O |
| H | H | Br | H | Me | Me | Et | O |
| H | H | Me | H | Et | Bu | Me | O |
| H | H | Me | H | Me | Et | Bu | O |
| OEt | OEt | H | H | Et | Cyclohexyl | Me | O |
| OBu | H | H | H | Me | Et | Et | O |
| H | H | Ph | Ph | Me | Me | C₆H₁₃ | O |
| OMe | H | H | Br | Me | Me | Me | O |
| SEt | H | H | Cl | Me | H | Bu | O |

Specific examples of Compounds (16) and (17) shown in Schemes 3 and 4 are as follows.

**[Table 2]**

| R₁, R₄ | | R₂, R₃ | | R₅, R₆ | | R₈, R₉ | |
|---|---|---|---|---|---|---|---|
| H | H | H | H | Me | Me | Me | Me |
| H | H | H | H | Me | Et | Me | Et |
| H | H | H | H | Me | Me | Et | Et |
| OMe | H | H | H | Et | Et | Et | Et |
| OMe | OMe | H | H | Et | Bu | Et | Bu |
| H | H | t-Bu | H | Me | Me | Me | Me |
| H | H | Cl | Cl | Me | Me | Et | Et |
| CF₃ | H | H | H | Me | Me | Cyclohexyl | Cyclohexyl |
| F | F | F | F | Bu | Bu | Bu | Bu |
| H | H | Me | H | Me | Me | H | H |
| SMe | H | H | Br | Bu | Bu | Et | H |
| SPh | H | H | H | Me | H | Me | H |
| Cl | Cl | Cl | Cl | Et | Cyclobutyl | Et | Cyclobutyl |
| OBu | OBu | H | H | Et | H | Me | Me |
| OMe | H | H | Cl | Me | Me | Me | Me |
| NO₂ | H | H | H | Me | Me | Me | Me |
| H | H | NO₂ | H | Me | Bu | Me | Me |
| Br | H | H | H | Et | C₆H₁₃ | Et | C₆H₁₃ |
| Br | Br | Cl | Cl | Et | Bu | Et | Bu |

Specific examples of Compounds (21) and (22) shown in Schemes 5 and 6 are as follows.

**[Table 3]**

| R₁, R₄ | | R₂, R₃ | | R₅, R₆ | | R₁₀ | R₁₁ | R₁₂ | R₁₃ | R₁₄ |
|---|---|---|---|---|---|---|---|---|---|---|
| H | H | H | H | Me | Me | H | H | H | H | H |
| H | H | H | H | Me | Et | H | H | H | H | H |
| H | H | H | H | Me | Me | Me | H | H | H | H |
| H | H | H | H | Me | Me | C₆H₁₃ | H | H | H | H |
| NO₂ | H | H | H | Et | H | Me | H | H | H | H |
| H | H | NO₂ | H | Et | Bu | Me | H | Cl | H | H |
| H | H | H | H | Me | Me | H | H | F | H | H |
| H | H | H | H | Et | Cyclohexyl | Bu | Br | Br | Br | Br |
| H | H | H | H | Me | Et | H | H | H | H | H |
| H | H | OEt | H | Me | Me | H | H | H | H | H |
| F | F | F | F | Me | Me | Cyclohexyl | H | H | H | H |
| H | H | Cl | Cl | Me | Me | Bu | H | H | H | H |
| CF₃ | H | H | H | Et | H | H | H | Br | H | H |
| OPh | OPh | H | H | Me | Me | Et | H | H | H | H |
| OMe | OMe | H | H | Et | Et | H | H | H | H | H |
| H | H | SEt | H | C₂H₄OC₂H₅ | C₂H₄OC₂H₅ | Me | H | H | H | H |
| Cl | Cl | Cl | Cl | Me | H | Me | H | Cl | H | H |

The isoindoline compound used in the coloring composition of the present embodiment that is further refined as necessary to a desired particle size by a method such as acid pasting, solvent salt milling, or dry milling may be used.

When the isoindoline compound (referred to as a pigment in the following description) is refined by acid pasting, the pigment is dissolved in concentrated sulfuric acid and mixed with a large excess amount of water, and fine pigment particles are precipitated. Then, filtration and washing with water are repeated and drying is performed to obtain refined pigment particles.

An acid pasting method is not particularly limited, and examples thereof include a method in which the pigment is dissolved in 5 to 30 times by mass of 98% sulfuric acid, and the obtained sulfuric acid solution is mixed with 5 to 30 times by mass of water. The temperature at which the pigment is dissolved in sulfuric acid may be set such that reactions such as decomposition and sulfonation of raw materials occur. Although not particularly limited, the temperature during dissolving is preferably, for example, 3 to 40°C. In addition, a method of mixing a sulfuric acid solution for a pigment and water and conditions such as a mixing temperature are not particularly limited. In many cases, when mixing at a low temperature rather than a high temperature, the precipitated pigment particles tend to be better refined. Therefore, the temperature during mixing is preferably, for example, 0°C to 60°C. Water used during mixing may be industrially usable water. However, in order to reduce an increase in temperature during precipitation, water cooled in advance is preferable.

A method of mixing a sulfuric acid solution and water is not particularly limited, and any mixing method may be used as long as the pigment can be completely precipitated. For example, pigment particles can be precipitated by a method of injecting a sulfuric acid solution into ice water prepared in advance, a method of continuously injecting a sulfuric acid solution into running water using a device such as an aspirator, or the like.

The slurry obtained by the above method is filtered and washed to remove acid components and then dried and crushed to obtain a pigment with a particle size adjusted as desired. When the slurry is filtered, the slurry in which a sulfuric acid solution and water are mixed may be directly filtered, but if the slurry has poor filterability, the slurry may be heated and stirred and then filtered. In addition, the slurry may be neutralized with a base and then filtered.

When the pigment is refined by solvent salt milling, a clay-like mixture composed of at least three components including a pigment, a water-soluble inorganic salt and a water-soluble solvent is strongly kneaded using a kneader or the like. The mixture after kneading is put into water, and stirred with various stirrers to form a slurry. The water-soluble inorganic salt and the water-soluble solvent are removed by filtering the obtained slurry. When the above forming into a slurry and filtration and washing with water are repeated, a refined pigment can be obtained.

As the water-soluble inorganic salt, sodium chloride, sodium sulfate, potassium chloride, or the like can be used. These inorganic salts are used in a range of 1 time by mass or more, or preferably 20 times by mass or less of the organic pigment. When the amount of the inorganic salt is 1 time by mass or more, the pigment can be sufficiently refined. In addition, when the amount of the inorganic salt is 20 times by mass or less, this is preferable in consideration of the productivity because a large amount of labor for removing the water-soluble inorganic salt and the water-soluble solvent after kneading is unnecessary, and at the same time, the amount of the pigment that can be processed at one time is not reduced.

In the above pigment refining method, heat is often generated during kneading. Therefore, in consideration of safety, it is preferable to use a water-soluble solvent having a boiling point of about 120 to 250°C. Specific examples of such water-soluble solvents include 2-(methoxymethoxy)ethanol, 2-butoxyethanol, 2-(isopentyloxy)ethanol, 2-(hexyloxy)ethanol, ethylene glycol, diethylene glycol, diethylene glycol monomethyl ether, diethylene glycol monoethyl ether, diethylene glycol monobutyl ether, triethylene glycol, triethylene glycol monomethyl ether, liquid polyethylene glycols, 1-methoxy-2-propanol, 1-ethoxy-2-propanol, dipropylene glycol, dipropylene glycol monomethyl ether, dipropylene glycol monoethyl ether, and low-molecular-weight polypropylene glycols.

When the pigment is refined by dry milling, the pigment is refined by dry crushing using various crushing machines. In this method, crushing proceeds through collisions or friction between crushing media. The device used for dry milling is not particularly limited, and specific examples thereof include a ball mill, an attritor, and a vibration mill, which are dry crushing devices in which a crushing medium such as beads is incorporated. When dry crushing is performed using these devices, as necessary, the inside of the crushing container may be depressurized or filled with an inert gas such as nitrogen gas. In addition, after dry milling, the above solvent salt milling and a stirring treatment in the solvent may be performed.

### [Dispersion medium]

In this specification, the dispersion medium includes a resin and a solvent. The dispersion medium can be appropriately selected depending on applications of the coloring composition. Examples of applications of the coloring composition include a colorant for plastics, a plastic molding material, a plastic molded product, a toner, a pigment dispersion, a paint, a printing ink, and an inkjet ink. The coloring composition can be obtained by mixing and dispersing the pigment containing Isoindoline Compound (1) and a dispersion medium according to a method known in the art.

For the ratio between Isoindoline Compound (1) in the coloring composition of the present embodiment and the dispersion medium, there is preferably 0.001 to 0.7 times and more preferably 0.01 to 0.6 times of Isoindoline Compound (1) with respect to 100 parts by mass of the dispersion medium.

The resin as the dispersion medium may be a polyolefin resin, a polyester resin, a styrene copolymer, an acrylic resin, or a modified resin thereof. Specific examples thereof include polyolefin resins such as polyethylene such as high density polyethylene (HDPE), linear low density polyethylene (L-LDPE), and low density polyethylene (LDPE), and polypropylene; polyester resins such as polyethylene terephthalate; styrene copolymers such as styrene-p-chloro styrene copolymers, styrene-vinyl toluene copolymers, styrene-vinyl naphthalene copolymers, styrene-acrylic acid ester copolymers, styrene-methacrylate copolymers, styrene-α-chloromethyl methacrylate copolymers, styrene-acrylonitrile copolymers, styrene-vinyl methyl ether copolymers, styrene-vinyl ethyl ether copolymers, styrene-vinyl methyl ketone copolymers, styrene-butadiene copolymers, styrene-isoprene copolymers, and styrene-acrylonitrile-indene copolymers; acrylic resins such as acrylic resins and methacrylic resins; and polyvinyl chloride, phenolic resins, naturally modified phenolic resins, natural resin-modified maleic acid resins, polyvinyl acetate, silicone resins, polyurethane, polyamide resins, epoxy resins, xylene resins, polyvinyl butyral, terpene resins, coumarone indene resins, alkyd resins, amino resins, epoxy resins, petroleum resins, and modified resins thereof.

As the dispersion medium, the solvent may be water, an aromatic hydrocarbon-based organic solvent, or an aliphatic hydrocarbon-based organic solvent. Specific examples thereof include water such as deionized water and distilled water; aromatic hydrocarbon-based organic solvents such as toluene and xylene; and aliphatic hydrocarbon-based organic solvents such as acetate-based organic solvents, ketone-based organic solvents, and alcohol-based organic solvents. More specific examples thereof include acetate-based organic solvents such as butyl acetate and methyl acetate; ketone-based organic solvents such as methyl ethyl ketone and methyl isobutyl ketone; and alcohol-based organic solvents such as ethanol, n-propanol, isopropanol, n-butanol, isobutanol, ethylene glycol, diethylene glycol, triethylene glycol, propylene glycol, methoxy propanol, methoxy butanol, 1-ethoxy-2-propanol, 2-(methoxymethoxy)ethanol, 2-butoxy ethanol, 2-(isopentyloxy)ethanol, 2-(hexyloxy)ethanol, glycerin, diethylene glycol, diethylene glycol monomethyl ether, diethylene glycol monoethyl ether, diethylene glycol monobutyl ether, triethylene glycol, triethylene glycol monomethyl ether, liquid polyethylene glycols, dipropylene glycol, dipropylene glycol monomethyl ether, dipropylene glycol monoethyl ether, liquid polypropylene glycol, butyl glycol, and butyl diglycol.

Hereinafter, the coloring composition will be described in more detail with reference to specific applications.

### (Coloring composition for plastics)

In one embodiment, the coloring composition can be suitably used as a plastic coloring application. In one embodiment, the coloring composition for plastics includes a pigment containing Isoindoline Compound (1) and a resin. The coloring composition for plastics is required to have durability with which discoloration does not occur even after heating at a high temperature of, for example, 300°C. On the other hand, according to the coloring composition containing Isoindoline Compound (1), excellent weather resistance and heat resistance can be easily obtained.

In the coloring composition for plastics, specific examples of resins that can be used as dispersion media include homopolymers and copolymers using ethylene, propylene, butylene, styrene or the like as a monomer component. More specific examples thereof include polyolefin resins such as polyethylene such as high density polyethylene (HDPE), linear low density polyethylene (L-LDPE), and low density polyethylene (LDPE), polypropylene, and polybutylene. Specific examples of other beneficial resins include polyester resins such as polyethylene terephthalate, polyamide resins such as nylon 6 and nylon 66, polystyrene resins, and thermoplastic ionomer resins.

In one embodiment, the resin preferably includes at least one of a polyolefin resin and a polyester resin. In one embodiment, the resin preferably includes at least a polyolefin resin. When a polyolefin resin is used, a more remarkable effect tends to be obtained in improving properties such as dispersibility, weather resistance, and heat resistance in the coloring composition.

As one embodiment of the coloring composition for plastics, a so-called masterbatch or a pellet form called a collar chip (hereinafter referred to as a colorant for plastics), which contains a high concentration of Isoindoline Compound (1) that functions as a pigment may be exemplified.

### (Plastic molding material)

In another embodiment, a resin is additionally added to the colorant for plastics containing a high concentration of Isoindoline Compound (1), and thus a coloring composition for forming a plastic molded product (hereinafter referred to as a plastic molding material) can be obtained. Therefore, one embodiment may be a plastic molding material containing the colorant for plastics. In the plastic molding material, the resin (base resin) to be added to the colorant for plastics is not particularly limited, and can be selected from resins known in the art depending on applications of the plastic molded product. The same resin as the resin used for the colorant for plastics may be used.

As the dispersion medium for the plastic molding material, the same dispersion media exemplified in the coloring composition for plastics can be used. Examples thereof include homopolymers and copolymers using ethylene, propylene, butylene, styrene or the like as a monomer component. More specific examples thereof include polyolefin resins such as polyethylene such as high density polyethylene (HDPE), linear low density polyethylene (L-LDPE), and low density polyethylene (LDPE), polypropylene, and polybutylene. Specific examples of other beneficial resins include polyester resins such as polyethylene terephthalate, polyamide resins such as nylon 6 and nylon 66, polystyrene resins, and thermoplastic ionomer resins.

In one embodiment, the polyolefin resin can be suitably used as a dispersion medium for the plastic molding material. The polyolefin resin used preferably has a melt flow rate (MFR, so-called melt viscosity) in a range of 0.001 to 30. When the MFR is 0.001 or more, the molding processability of the plastic molding material is good and weld marks or flow marks are unlikely to occur in the molded product. On the other hand, when the MFR is 30 or less, excellent mechanical properties can be easily obtained in the molded product. In particular, when high density polyethylene (HDPE) is used, the MFR is preferably in a range of 0.005 to 10. In addition, when low density polyethylene (LDPE) or polypropylene, polybutene is used, the MFR is preferably in a range of 0.005 to 20.

The colorant for plastics and the plastic molding material may further contain other additive components as long as desired effects are not impaired and there is no problem in hygiene.

Examples of additive components include wax or its derivatives; heavy metal deactivators, alkali metals, alkaline earth metals or zinc metal soaps; hydrotalcites; antistatic agents composed of nonionic surfactants, cationic surfactants, anionic surfactants, amphoteric surfactants and the like; halogen-based, phosphorus-based or metal oxide flame retardants; lubricants such as ethylene bisalkyl amide; antioxidants and ultraviolet absorbing agents; processing aids; fillers; and various additives for known polymers. In addition, in order to adjust the color tone, as necessary, an inorganic pigment and an organic pigment other than Isoindoline Compound (1) may be added.

### (Plastic molded product)

In one embodiment, the molding method for obtaining a colorant for plastics such as collar chip and a plastic molded product composed of a plastic molding material is not particularly limited. Examples of molding methods include blow molding, inflation molding, extrusion molding, Engel molding, and vacuum molding. According to the improved dispersibility and heat resistance of Isoindoline Compound (1) contained in the colorant for plastics and the plastic molding material, a plastic molded product that favorably maintains a desired hue without causing discoloration even after heating is performed at a high temperature during molding is obtained.

### (Toner)

In one embodiment, the coloring composition can be suitably used in toner applications. The coloring composition for toner contains a pigment containing Isoindoline Compound (1) and a dispersion medium (binding resin). When the coloring composition is used for the toner application, it is preferable that the coloring composition not discolor in a usage environment in which, for example, heating and irradiation with UV light occur. On the other hand, since the coloring composition containing Isoindoline Compound (1) has excellent weather resistance and heat resistance, discoloration is inhibited, and a desired hue can be maintained.

The toner may be either a dry toner or a wet toner. For example, the dry toner can be produced by melt-kneading a coloring composition for toner containing Isoindoline Compound (1) and a dispersion medium (binding resin), performing cooling and then performing a crushing and classification step, and additionally performing a post-treatment step of adding and mixing additional components, as necessary. The toners can be produced using the coloring composition for toner according to other methods known in the art.

The dispersion medium (binding resin) constituting the coloring composition for toner is not particularly limited, and examples thereof include styrene-p-chloro styrene copolymers, styrene-vinyl toluene copolymers, styrene-vinyl naphthalene copolymers, styrene-acrylic acid ester copolymers, styrene-methacrylate copolymers, styrene-α-chloromethyl methacrylate copolymers, styrene-acrylonitrile copolymers, styrene-vinyl methyl ether copolymers, styrene-vinyl ethyl ether copolymers, styrene-vinyl methyl ketone copolymers, styrene-butadiene copolymers, styrene-isoprene copolymers, styrene-acrylonitrile-indene copolymers, polyvinyl chloride, phenolic resins, naturally modified phenolic resins, natural resin-modified maleic acid resins, acrylic resins, methacrylic resins, polyvinyl acetate, silicone resins, polyester resins, polyurethane, polyamide resins, furan resins, epoxy resins, xylene resins, polyvinyl butyral, terpene resins, coumarone indene resins, and petroleum resins.

In one embodiment, the binding resin preferably contains a polyester resin or a styrene copolymer, and preferably contains at least polyester resin. Among the dispersion media (binding resins), Isoindoline Compound (1) has particularly excellent suitability for polyester resins. Therefore, in one embodiment, the coloring composition for toner preferably contains Isoindoline Compound (1) and a binding resin including a polyester resin. In such a coloring composition for toner, the isoindoline compound is uniformly and finely dispersed in the binding resin including a polyester resin, and thus it is possible to provide a high-quality toner.

The polyester resin used as the binding resin may be a resin obtained by reacting an alcohol component with an acid component. As the alcohol component constituting the polyester resin, multivalent alcohols such as ethylene glycol, 1,2-propylene glycol, 1,3-propylene glycol, 1,3-butanediol, 1,4-butanediol, 2,3-butanediol, 1,4-butenediol, diethylene glycol, triethylene glycol, 1,5-pentanediol, 1,6-hexanediol, neopentyl glycol, 2-ethyl-1,3-hexanediol, bisphenol A, hydrogenated bisphenol A, 1,4-bis(hydroxymethyl)cyclohexane, glycerol, diglycerol, sorbitol, sorbitan, butanetriol, trimethylolethane, trimethylolpropane, pentaerythritol, dipentaerythritol, and tripentaerythritol, and bisphenol derivatives represented by the following Formula (D) can be used. These may be used alone or two or more thereof may be used in combination.

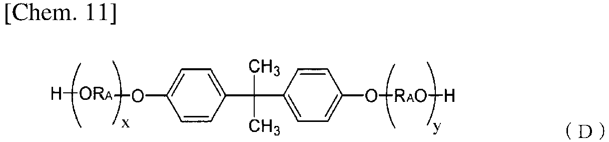

In the formula, R_{A} is an ethylene group or a propylene group, x and y are each an integer of 1 or more, and x+y is 2 to 10.

The acid component constituting the polyester resin may be a divalent carboxylic acid. Examples of divalent carboxylic acids include aromatic dicarboxylic acids such as phthalic acid, terephthalic acid, isophthalic acid, and phthalic anhydride or anhydrides thereof; aliphatic dicarboxylic acids such as succinic acid, adipic acid, sebacic acid, and azelaic acid or anhydrides thereof; succinic acids substituted with an alkyl group having 16 to 18 carbon atoms or anhydrides thereof; and aliphatic unsaturated dicarboxylic acids such as fumaric acid, maleic acid, citraconic acid, and itaconic acid or anhydrides thereof.

As the acid component, a trivalent or higher carboxylic acid effective as a cross-linking component may be used. Examples thereof include trimellitic acid, pyromellitic acid, naphthalene tricarboxylic acid, butane tricarboxylic acid, hexane tricarboxylic acid, benzophenone tetracarboxylic acid, tetra(methylenecarboxyl)methane, octanetetracarboxylic acid, and benzophenone tetracarboxylic acid, and anhydrides thereof.

As the acid component, the exemplified compounds may be used alone or two or more thereof may be used in combination.

The polyester resin may be either a homopolyester or a copolyester obtained by reacting an alcohol component with an acid component. The polyester resins may be used alone or two or more thereof may be used in combination.

In one embodiment, in consideration of the offset resistance and low temperature fixability with respect to the toner, the weight average molecular weight (Mw) of the polyester resin is preferably 5,000 or more, and more preferably in a range of 10,000 to 1,000,000, and still more preferably in a range of 20,000 to 100,000. The weight average molecular weight is a molecular weight measured through gel permeation chromatography (GPC). When the weight average molecular weight is 5,000 or more, excellent offset resistance can be easily obtained in the toner. In addition, when the weight average molecular weight is 1,000,000 or less, excellent fixability can be easily obtained.

In one embodiment, the acid value of the polyester resin is preferably in a range of 10 to 60 mg KOH/g and more preferably in a range of 15 to 55 mg KOH/g. When the acid value is adjusted to 10 mg KOH/g or more, the release of the mold release agent can be easily minimized. In addition, when the acid value is adjusted to 60 mg KOH/g or less, it is possible to minimize an increase in hydrophilicity of the resin, and it is possible to prevent a decrease in the image density in a high humidity environment.

In one embodiment, the hydroxyl value of the polyester resin is preferably 20 mg KOH/g or less and more preferably 15 mg KOH/g or less. When the hydroxyl value is adjusted to 20 mg KOH/g or less, it is possible to minimize an increase in hydrophilicity of the resin, and it is possible to minimize a decrease in the image density in a high humidity environment.

In addition, in consideration of preventing toner aggregation, the glass transition temperature (Tg) of the polyester resin measured by a differential scanning calorimeter (device: DSC-6, commercially available from Shimadzu Corporation) is preferably 50 to 70°C and more preferably 50 to 65°C.

A charge control agent can be added to the coloring composition for toner, as necessary. When a charge control agent is used, a toner having a stable charge amount can be easily obtained. The charge control agent added to the coloring composition for toner may be a conventionally known positive or negative charge control agent.

In one embodiment, when the toner is a positively chargeable toner, specific examples of positive charge control agents that can be used include nigrosine dyes, triphenylmethane dyes, organic tin oxides, quaternary ammonium salt compounds, and styrene/acrylic polymers copolymerized with styrene/acrylic resins using a quaternary ammonium salt as a functional group. Among these, a quaternary ammonium salt compound is preferable. Specific examples of quaternary ammonium salt compounds that can be used include salt-forming compounds composed of quaternary ammonium salts, organic sulfonic acids or molybdic acids. As the organic sulfonic acid, naphthalene sulfonic acid is preferably used.

On the other hand, when the toner is a negatively charged toner, specific examples of negative charge control agents that can be used include metal complexes of monoazo dyes, styrene/acrylic polymers copolymerized with styrene/acrylic resins using sulfonic acid as a functional group, metal salt compounds of aromatic hydroxycarboxylic acids, metal complexes of aromatic hydroxycarboxylic acids, phenolic condensates, and phosphonium compounds. As the aromatic hydroxycarboxylic acid, salicylic acid, 3,5-di-tert-butylsalicylic acid, 3-hydroxy-2-naphthoic acid, or 3-phenyl-salicylic acid is preferable. In addition, examples of metals used in the metal salt compound include zinc, calcium, magnesium, chromium, and aluminum.

In addition, a mold release agent can be used for the coloring composition for toner. Examples of mold release agents include hydrocarbon-based waxes such as polypropylene wax, polyethylene wax, and Fischer-Tropsch wax, synthetic ester waxes, and natural ester-based waxes such as carnauba wax and rice wax.

In addition, as necessary, external additives such as a lubricant, a fluidizing agent, a polishing agent, a conductivity-imparting agent, and an image peeling preventing agent may be added to the coloring composition for toner. These external additives can be selected from among conventionally known external additives used for producing the toner.

Specific examples of such external additives are as follows.

The lubricant may be polyvinylidene fluoride, zinc stearate or the like. The fluidizing agent may be silica, aluminum oxide, titanium oxide, silicon-aluminum co-oxides, and silicon-titanium co-oxides produced by a drying method or a wet method, and hydrophobized products thereof. The polishing agent may be silicon nitride, cerium oxide, silicon carbide, strontium titanate, tungsten carbide, calcium carbonate, and hydrophobized products thereof. The conductivity-imparting agent may be tin oxide.

In the coloring composition for toner, among the fluidizing agents exemplified above, at least one of hydrophobized silica, silicon-aluminum co-oxide, and silicon-titanium co-oxide fine powder is preferably used. Examples of hydrophobic treatment methods for these fine powders include treatments using a silane coupling agent such as silicon oil, tetramethyldisilazane, dimethyldichlorosilane, or dimethyldimethoxysilane.

In one embodiment, the toner formed from the coloring composition for toner can be used as one-component developing agent. In another embodiment, the toner can also be used as a two-component developing agent. When the toner is used as a two-component developing agent, the coloring composition for toner further contains a carrier. The carrier used for the toner used as a two-component developing agent may be a material known in the art.

Examples of materials that can be used as carriers include magnetic powders such as iron powder, ferrite powder, and nickel powder, and those whose surfaces are treated with a resin or the like. Examples of resins that cover the surface of the carrier include styrene-acrylic acid ester copolymers, styrene-methacrylate copolymers, acrylic acid ester copolymers, methacrylate copolymers, fluorine-containing resins, silicone-containing resins, polyamide resins, ionomer resins, polyphenylene sulfide resins, and mixtures thereof. Among these, a silicone-containing resin is particularly preferable because the formation of spent toner is little. The weight average particle size of these carriers is preferably in a range of 30 to 100 µm.

### (Pigment dispersion)

In one embodiment, the coloring composition may be a pigment dispersion that can be suitably used for producing paints, printing inks, inkjet inks and the like. That is, in one embodiment, the coloring composition may be a pigment containing Isoindoline Compound (1) and a pigment dispersion containing a resin and a solvent as a dispersion medium.

It is preferable that the coloring composition composed as a pigment dispersion be less likely to increase the viscosity after the dispersion treatment and have excellent dispersion stability of the pigment. For example, when it is assumed that the composition is used as a coating-forming material such as a paint or a printing ink, it is preferable that the composition be able to form a coating having excellent light resistance and have favorable initial viscosity and storage stability.

However, since the conventional isoindoline pigment has low dispersibility of the pigment itself, a dispersing agent of a polymer compound is used in many cases. Therefore, the pigment dispersion using a conventional isoindoline pigment tends to have high initial viscosity and poor storage stability. On the other hand, when a pigment dispersion is formed using a coloring composition containing Isoindoline Compound (1), it is possible to provide a pigment dispersion having excellent dispersibility, initial viscosity and storage stability.

### (Paint and printing ink)

In one embodiment, the coloring composition can be used as a pigment dispersion to form a composition constituting a paint or printing ink. The usage form of paints and printing inks is not particularly limited, and may be a form for automobiles, wood, metals and the like. The printing ink may be an offset printing ink, a flexographic printing ink, a gravure printing ink, a radiation curing type ink, a color filter ink, an inkjet printer ink to be described below or the like.

The solvent as a dispersion medium may be water and/or an organic solvent, and can be appropriately selected depending on the form of a desired paint or printing ink. In one embodiment, the pigment dispersion contains a pigment containing Isoindoline Compound (1), and a resin and water as a dispersion medium. In another embodiment, the pigment dispersion contains a pigment containing Isoindoline Compound (1), and a resin, water, and an organic solvent as a dispersion medium. In the above embodiment, when a solvent is used as the dispersion medium, the viscosity can be adjusted to a desired value depending on applications of the pigment dispersion.

In the above embodiment, the resin as a dispersion medium preferably includes a binder resin. For example, the binder resin can be selected from among an acrylic resin, an alkyd resin, a polyester resin, an amino resin, an epoxy resin, and modified resins thereof. For the resin, additionally, it is preferable to use a curing agent or cross-linking agent for an amino resin, an isocyanate compound, a blocked isocyanate compound, a polyamide resin, a melamine resin or the like in combination.

In the case of non-aqueous paints or printing inks, toluene, xylene, butyl acetate, methyl acetate, methyl ethyl ketone, methyl isobutyl ketone, butyl alcohol, aliphatic hydrocarbon or the like can be used as the solvent. In addition, solvents generally used in the paint or printing ink in the art can also be used.

In the case of aqueous paints or printing inks, water or water-dilutable monovalent or divalent alcohols, and glycol can be used as the solvent. Specific examples thereof include ethanol, n-propanol, isopropanol, n-butanol, isobutanol, ethylene glycol, diethylene glycol, triethylene glycol, propylene glycol, and glycerin. Water-dilutable monoether derived from multivalent alcohols can also be used as the solvent. Specific examples include methoxy propanol and methoxy butanol. In addition, for example, water-dilutable glycol ethers such as butyl glycol and butyl diglycol can be used. These may be used alone or two or more thereof may be used in combination.

In one embodiment, the paint or printing ink may be composed of a pigment containing Isoindoline Compound (1), a binder resin, and a solvent, and as necessary, other components may be added.

For example, in one embodiment, metal flakes, micas, and coated glass flakes having an average thickness of 0.5 to 10 µm and an average particle size of 5 to 50 µm may be added as a glittering material. Examples of metal flakes include aluminum flakes and gold powder. Examples of micas include general micas and other coated micas. Examples of coated glass flakes include glass flakes coated with metal oxides such as titanium oxide.

The amount of the glittering material added is preferably 0.1 to 10 mass% with respect to the total mass of the paint or the printing ink containing a pigment containing Isoindoline Compound (1), a binder resin, and a solvent. Here, in addition, other coloring pigments generally used in the art and various additives may be added as necessary. A method of producing a paint or printing ink, a coating method, and a drying method are not particularly limited, and methods well-known in the art can be applied.

### (Aqueous pigment dispersion)

In one embodiment, the aqueous pigment dispersion contains Isoindoline Compound (1), and a resin and a solvent as a dispersion medium. Examples of resins constituting the aqueous pigment dispersion include styrene-acrylic acid copolymers, acrylic acid-acrylic acid acrylic ester copolymers, styrene-acrylic acid-acrylic acid alkyl ester copolymers, styrene-methacrylic acid copolymers, styrene-methacrylic acid-acrylic acid acrylic ester copolymers, styrene-α-methyl styrene-acrylic acid copolymers, styrene-α-methyl styrene-acrylic acid-acrylic acid alkyl ester copolymers, polyacrylic acid, polymethacrylic acid, vinyl naphthalene-acrylic acid copolymer salts, styrene-maleic acid copolymer salts, maleic acid-maleic anhydride copolymer salts, vinyl naphthalene-maleic acid copolymer salts, and polyester-modified acrylic acid polymers. These may be used alone or two or more thereof may be used in combination.

In addition, when the water-soluble resin is prepared, a resin obtained by copolymerizing monomers such as acrylonitrile, vinyl acetate, acrylamide, vinyl chloride, vinylidene chloride, ethylene, and hydroxyethyl acrylate may be used. These exemplified resins may be used alone or two or more thereof may be used in combination.

The resin can be used in a range of 0.1 to 30 mass%, preferably 1 to 20 mass% based on the total mass of the aqueous pigment dispersion.

The solvent as the dispersion medium used for producing an aqueous pigment dispersion is not particularly limited, and an aqueous solvent is preferable. Among these, deionized water or distilled water from which metal ions and the like have been removed is preferable. The content of the aqueous solvent in the aqueous pigment dispersion is not particularly limited, and is preferably 30 to 95 mass%.

Water and/or a water-soluble organic solvent (hereinafter referred to as a water-soluble solvent) can be used as the aqueous solvent. The water-soluble solvent is not particularly limited, and examples thereof include 2-(methoxymethoxy)ethanol, 2-butoxy ethanol, 2-(isopentyloxy)ethanol, 2-(hexyloxy)ethanol, diethylene glycol, diethylene glycol monomethyl ether, diethylene glycol monoethyl ether, diethylene glycol monobutyl ether, triethylene glycol, triethylene glycol monomethyl ether, liquid polyethylene glycols, 1-methoxy-2-propanol, 1-ethoxy-2-propanol, dipropylene glycol, dipropylene glycol monomethyl ether, dipropylene glycol monoethyl ether, and liquid polypropylene glycol. These may be used alone or two or more thereof may be used in combination.

The aqueous pigment dispersion may further contain a surfactant, as necessary. The surfactant may be an anionic or nonionic, and a compound that can function as a dispersing agent can be used without particular limitation.

Examples of anionic surfactants include fatty acid salt, alkyl sulfate ester salt, alkylaryl sulfonate, alkylnaphthalene sulfonate, dialkyl sulfonate, dialkyl sulfonate, dialkylsulfosuccinate, alkyl phosphate, polyoxyethylene alkyl ether sulfate, polyoxyethylene alkylaryl ether sulfate, naphthalene sulfonic acid formarin condensate, polyoxyethylene alkyl phosphate ester salt, glycerol volatile fatty acid ester, and polyoxyethylene glycerol fatty acid ester.

Examples of nonionic surfactants include polyoxyethylene alkyl ether, polyoxyethylene alkylaryl ether, polyoxyethylene oxypropylene block copolymer, sorbitan fatty acid ester, polyoxyethylene sorbitan fatty acid ester, polyoxyethylene sorbitol fatty acid ester, glycerin fatty acid ester, and polyoxyethylene fatty acid ester polyoxyethylene alkylamine.

These surfactants may be used alone or two or more thereof may be used in combination.

The content of the surfactant based on the total mass of the aqueous pigment dispersion is preferably 0.3 to 20 mass% and more preferably 1 to 10 mass%.

The aqueous pigment dispersion can be prepared by dispersing a mixture of the raw materials using various dispersers. In addition, as necessary, in addition to the above raw materials, various additives may be additionally added for a dispersion treatment. In the preparation of the aqueous pigment dispersion, the order of adding respective raw materials or an addition method is not particularly limited, and a method known in the art can be applied.

In addition, in order to produce an aqueous pigment dispersion, as necessary, additives such as a preservative, a pH adjusting agent, an antifoaming agent, and a moistening agent can be used.

The preservative is used to prevent the growth of mold and bacteria in the aqueous pigment dispersion. The type of the preservative is not particularly limited, and examples thereof include sodium dehydroacetate, sodium benzoate, and sodium pyridinethion-1-oxide, zinc pyridinethion-1-oxide, 1,2-benzisothiazolin-3-one, and 1-benzisothiazolin-3-one amine salts. These are preferably used in a range of 0.1 to 2 mass% based on the total mass of the pigment dispersion.

The pH adjusting agent is used to adjust the pH of the aqueous pigment dispersion to a desired value. The type of the pH adjusting agent is not particularly limited, and examples thereof include various amines, inorganic salts, ammonia, and various buffer solutions.

The antifoaming agent is used to prevent formation of bubbles when the aqueous pigment dispersion is produced. The type of the antifoaming agent is not particularly limited, and commercially available products can be used. Examples thereof include SURFYNOL 104E, SURFYNOL 104H, SURFYNOL 104A, SURFYNOL 104BC, SURFYNOL 104DPM, SURFYNOL 104PA, SURFYNOL 104PG-50, SURFYNOL 420, SURFYNOL 440, SURFYNOL 465, SURFYNOL 485, and SURFYNOL PSA-336 (all commercially available from Nissin Chemical Co., Ltd.), and ADDITOLVXW 6211, ADDITOLVXW4973, ADDITOL VXW6235, ADDITOLXW375, ADDITOL XW376, ADDITOLVXW6381, ADDITOL VXW6386, ADDITOLVXW6392, ADDITOL VXW6393, ADDITOLVXW6399, ADDITOL XW6544, and the like (all commercially available from Allnex).

The moistening agent is used to improve pigment dispersion in the aqueous pigment dispersion. A smooth coating can be easily obtained using the moistening agent. The type of the moistening agent is not particularly limited, and commercially available products can be used. Examples thereof include ADDITOLVXL6237N, ADDITOL XL260N, ADDITOLVXL6212, ADDITOL UVX7301/65, ADDITOL XW330, ADDITOL VXW6200, ADDITOLVXW6205, ADDITOL VXW6394, ADDITOLVXW6208, ADDITOL VXW6208/60, and ADDITOL VXW6374 (all commercially available from Allnex).

The disperser used for preparing an aqueous pigment dispersion is not particularly limited. Examples thereof include a horizontal sand mill, a vertical sand mill, an annular type bead mill, an attritor, a microfluitizer, a high-speed mixer, a homo mixer, a homogenizer, a ball mill, a paint shaker, roll mill, a mortar type mill, and an ultrasonic disperser. In addition, a disperser and a mixer generally used for preparing various dispersions in the art can be appropriately selected and used.

In addition, before dispersing with various dispersers, a treatment such as pre-dispersion using a kneading mixer such as a kneader and a 3-roll mill or solid dispersion using a 2-roll mill may be performed. In addition, after dispersing with various dispersers, a step of a post-treatment of storing for about several hours to 1 week in a heated state at 30 to 80°C and a post-treatment using an ultrasonic disperser or a collision type beadless disperser are effective in imparting dispersion stability to the pigment dispersion.

### (Aqueous inkjet ink)

One embodiment relates to an aqueous inkjet ink using the aqueous pigment dispersion. The aqueous inkjet ink can be produced by adding water, as necessary, a water-soluble solvent, a resin, and an additive to the aqueous pigment dispersion to form an ink.

In the aqueous inkjet ink, the content of the pigment containing Isoindoline Compound (1) is not particularly limited. For example, the content of the pigment based on the total mass of the aqueous inkjet ink is preferably 0.5 to 30 mass% and more preferably 1 to 15 mass%.

Water used for producing an aqueous inkjet ink is not particularly limited, and it is preferable to use deionized water or distilled water from which metal ions and the like have been removed. In addition, the content of water is not particularly limited, and is preferably 60 to 99 mass% based on the total mass of the aqueous pigment dispersion.

In order to prevent drying and solidification at a nozzle part in a printer head of the aqueous inkjet ink and realize stable ink discharge, as necessary, a water-soluble solvent can be used. The water-soluble solvent is not particularly limited, and examples thereof include ethylene glycol, diethylene glycol, propylene glycol, triethylene glycol, polyethylene glycol, glycerin, tetraethylene glycol, dipropylene glycol, ketone alcohol, diethylene glycol monobutyl ether, ethylene glycol monobutyl ether, ethylene glycol monoethyl ether, 1,2-hexanediol, N-methyl-2-pyrrolidone, substituted pyrrolidone, 2,4,6-hexanetriol, tetrafurfuryl alcohol, and 4-methoxy-4 methylpentanone. These may be used alone or two or more thereof may be used in combination. When such a water-soluble solvent is used, the content of the water-soluble solvent based on the total mass of the aqueous inkjet ink is preferably in a range of 1 to 50 mass%.

In the preparation of the aqueous inkjet ink, a resin can be used to impart ink fixability to an object to be printed. The resin that can be used is not particularly limited, and may be a water-soluble resin or a resin having excellent dispersibility in an aqueous medium. These resins may be used alone or two or more thereof may be used in combination.

Specific examples of resins include water-soluble resins such as acrylic resins, styrene-acrylic resins, polyester resins, polyamide resins, and polyurethane resin and oil-in-water emulsions thereof. Among these, an oil-in-water emulsion is preferable. An oil-in-water emulsion is preferably used because a low-viscosity aqueous inkjet ink and a recording object having excellent water resistance can be easily obtained.

The content of the resin based on the total mass of the aqueous inkjet ink is preferably 0.5 to 15 mass% and more preferably 1 to 10 mass%. When the content of the resin is 0.5 mass% or more, a sufficient effect can be easily obtained in fixing the coating component. On the other hand, when the content of the resin is 15 mass% or less, it is possible to minimize a decrease in the discharge stability of the aqueous inkjet ink. These resins can be used by neutralizing acid functional groups with the pH adjusting agent such as ammonia, various amines, and various inorganic alkalis, as necessary.

In the production of the aqueous inkjet ink, a drying accelerating agent, a penetrating agent, a preservative, a chelating agent, a pH adjusting agent, and the like may be used as other additives.

Alcohols such as methanol, ethanol, and isopropyl alcohol can be used in order to accelerate drying after printing with the aqueous inkjet ink. These alcohols may be used alone or two or more thereof may be used in combination. The content of these alcohols based on the total mass of the aqueous inkjet ink is preferably 1 to 50 mass%.

When the object to be printed is a permeable base material such as paper, various penetrating agents can be used in order to promote penetration of an ink into a base material and accelerate apparent drying. As the penetrating agent, glycol ethers such as diethylene glycol monobutyl ether and alkylene glycol, and the like, and surfactants such as polyethylene glycol monolauryl ether, sodium lauryl sulfate, sodium dodecylbenzenesulfonate, sodium oleate, and sodium dioctyl sulfosuccinate, exemplified as the water-soluble solvent, can be used. The amount of these used based on the total mass of the aqueous inkjet ink is 5 mass% or less, which is sufficiently effective. When the amount used is adjusted to 5 mass% or less, it is possible to inhibit the occurrence of problems such as print bleeding and paper slipping.

The preservative can be used to prevent the growth of mold and bacteria in the aqueous inkjet ink. The type of the preservative is not particularly limited, and examples thereof include sodium dehydroacetate, sodium benzoate, and sodium pyridinethion-1-oxide, zinc pyridinethion-1-oxide, 1,2-benzisothiazolin-3-one, and 1-benzisothiazolin-3-one amine salts. These are preferably used in a range of 0.05 to 1.0 mass% based on the total mass of the aqueous inkjet ink.

A chelating agent can be used in order to capture metal ions contained in the aqueous inkjet ink and prevent precipitation of insoluble substances in the nozzle part or the ink. The type of the chelating agent is not particularly limited, and examples thereof include ethylenediaminetetraacetic acid, ethylenediaminetetraacetic acid sodium salts, ethylenediaminetetraacetic acid diammonium salts, and ethylenediaminetetraacetic acid tetraammonium salts. The chelating agent is preferably used in a range of 0.005 to 0.5 mass% based on the total mass of the aqueous inkjet ink.

In addition, various pH adjusting agents can be used in order to adjust the pH of the aqueous inkjet ink to a desired value. The type of the pH adjusting agent is not particularly limited, and for example, various amines, inorganic salts, ammonia, and various buffer solutions can be used.

In the preparation of the inkjet ink, a method of mixing raw materials is not particularly limited, and mixing can be performed using a high-speed disperser, an emulsifier or the like in addition to a stirrer using general wings. In this case, the order of adding raw materials, a mixing method and the like are not particularly limited.

### Examples

Hereinafter, the present invention will be described in detail with reference to examples, but the present invention is not limited to the examples. Here, in the following, "parts" indicates "parts by mass," and "%" indicates "mass%."

### <Production of isoindoline compound>

### (Synthesis Example 1-1)

700 parts of water, 50 parts of 1,3-diiminoisoindoline, and 100 parts of 28% ammonia water were put in that order into a 3 L 4-neck flask including a reflux cooling tube, a dropping funnel, and a stirrer and the mixture was stirred. A solution prepared by dissolving 35.48 parts of 2-cyano-N-methylacetamide in 100 parts of water was added dropwise thereto using a dropping funnel for 30 minutes. The mixture was heated and stirred at 30°C until the raw material 1,3-diiminoisoindoline disappeared. The disappearance of the raw material was confirmed through ultra high performance liquid chromatography (UPLC). The reaction slurry was filtered off using a Buechner funnel to obtain a non-volatile content. The total amount of the non-volatile content was used in the following reaction.

A total amount of the non-volatile content obtained in the above preparation as a raw material, 1,200 parts of water, and 400 parts of 80% acetic acid were put into a 3 L 4-neck flask including a reflux cooling tube, a dropping funnel and a stirrer, and the mixture was stirred. 61.85 parts of 1,3-dimethylbarbituric acid was added thereto and the mixture was stirred at 85°C. Heating and stirring were performed until the non-volatile content used as a raw material disappeared. The disappearance of the raw material was confirmed through UPLC.

Then, cooling was performed to room temperature and washing with 2,000 parts of water was then performed three times to obtain a non-volatile content. The non-volatile content was dried in a hot air dryer at 80°C, and 114.45 parts (a yield of 91%) of Isoindoline Compound 1-1 was obtained.

### (Synthesis Example 1-2)

A reaction operation was performed in the same manner as in Synthesis Example 1-1 except that 40.56 parts of 2-cyano-N-ethylacetamide was used in place of 35.48 parts of 2-cyano-N-methylacetamide of Synthesis Example 1-1 and 72.97 parts of 1,3-diethyl barbituric acid was used in place of 61.85 parts of 1,3-dimethylbarbituric acid, and 122.02 parts (a yield of 87%) of Isoindoline Compound 1-2 was obtained.

### (Synthesis Example 1-3)

A reaction operation was performed in the same manner as in Synthesis Example 1-1 except that 25.59 parts of 2-cyano-N-methylacetamide of Synthesis Example 1-1 was used instead of 35.48 parts, 50 parts of 5-tert-butyl-1,3-diiminoisoindoline was used in place of 50 parts of 1,3-diiminoisoindoline, and 48.62 parts of 1-ethyl-3-methylbarbituric acid was used in place of 61.85 parts of 1,3-dimethylbarbituric acid, and 80.0 parts (a yield of 74%) of Isoindoline Compound 1-3 was obtained.

### (Synthesis Example 1-4)

A reaction operation was performed in the same manner as in Synthesis Example 1-1 except that 28.67 parts of 2-cyano-N-methylacetamide of Synthesis Example 1-1 was used instead of 35.48 parts, 50 parts of 5-chloro-1,3-diiminoisoindoline was used in place of 50 parts of 1,3-diiminoisoindoline, and 76.93 parts of 1,3-dibutylbarbituric acid was used in place of 61.85 parts of 1,3-dimethylbarbituric acid, and 104.91 parts (yield 78%) of Isoindoline Compound 1-4 was obtained.

### (Synthesis Example 1-5)

A reaction operation was performed in the same manner as in Synthesis Example 1-1 except that 27.08 parts of 2-cyano-N-methylacetamide of Synthesis Example 1-1 was used instead of 35.48 parts, 50 parts of 5-nitro-1,3-diiminoisoindoline was used in place of 50 parts of 1,3-diiminoisoindoline, and 47.21 parts of 1,3-dimethylbarbituric acid was used instead of 61.85 parts, and 51.76 parts (a yield of 48%) of Isoindoline Compound 1-5 was obtained.

### (Synthesis Example 1-6)

A reaction operation was performed in the same manner as in Synthesis Example 1-1 except that 27.08 parts of 2-cyano-N-methylacetamide of Synthesis Example 1-1 was used instead of 35.48 parts, 50 parts of 4-nitro-1,3-diiminoisoindoline was used in place of 50 parts of 1,3-diiminoisoindoline, and 47.21 parts of 1,3-dimethylbarbituric acid was used instead of 61.85 parts, and 52.84 parts (a yield of 49%) of Isoindoline Compound 1-6 was obtained.

### (Synthesis Example 1-7)

A reaction operation was performed in the same manner as in Synthesis Example 1-1 except that 18.20 parts of 2-cyano-N-methylacetamide of Synthesis Example 1-1 was used instead of 35.48 parts, 50 parts of 4,5,6,7-tetrachloro-1,3-diiminoisoindoline was used in place of 50 parts of 1,3-diiminoisoindoline, and 59.42 parts of 1,3-dicyclohexyl barbituric acid was used in place of 61.85 parts of 1,3-dimethylbarbituric acid, and 36.03 parts (a yield of 32%) of Isoindoline Compound 1-7 was obtained.

### (Synthesis Example 1-8)

A reaction operation was performed in the same manner as in Synthesis Example 1-1 except that 42.01 parts of 2-cyano-N-butyl acetamide was used in place of 35.48 parts of 2-cyano-N-methylacetamide of Synthesis Example 1-1, 50 parts of 4-methoxy-1,3-diiminoisoindoline was used in place of 50 parts of 1,3-diiminoisoindoline, and 46.64 parts of 1-methyl barbituric acid was used in place of 61.85 parts of 1,3-dimethylbarbituric acid, and 71.25 parts (a yield of 59%) of Isoindoline Compound 1-8 was obtained.

### (Synthesis Example 1-9)

A reaction operation was performed in the same manner as in Synthesis Example 1-1 except that 25.09 parts of 2-cyano-N-methylacetamide of Synthesis Example 1-1 was used instead of 35.48 parts, 50 parts of 5-thiomethyl-1,3-diiminoisoindoline was used in place of 50 parts of 1,3-diiminoisoindoline, and 39.81 parts of 1-methyl barbituric acid was used in place of 61.85 parts of 1,3-dimethylbarbituric acid, and 65.77 parts (a yield of 68%) of Isoindoline Compound 1-9 was obtained.

### (Synthesis Example 1-10)

A reaction operation was performed in the same manner as in Synthesis Example 1-1 except that 21.71 parts of 2-cyano-N-methylacetamide of Synthesis Example 1-1 was used instead of 35.48 parts, 50 parts of 4-phenoxy-1,3-diiminoisoindoline was used in place of 50 parts of 1,3-diiminoisoindoline, and 37.84 parts of 1,3-dimethylbarbituric acid was used instead of 61.85 parts, and 42.39 parts (a yield of 44%) of Isoindoline Compound 1-10 was obtained.

### (Synthesis Example 1-11)

A reaction operation was performed in the same manner as in Synthesis Example 1-1 except that 35.84 parts of 2-cyano-methyl acetate was used in place of 35.48 parts of 2-cyano-N-methylacetamide of Synthesis Example 1-1, and 89.54 parts (a yield of 71%) of Isoindoline Compound 1-11 was obtained.

### (Synthesis Example 1-12)

A reaction operation was performed in the same manner as in Synthesis Example 1-1 except that 26.50 parts of 2-cyano-methyl acetate was used in place of 35.48 parts of 2-cyano-N-methylacetamide of Synthesis Example 1-1, 50 parts of 5-bromo-1,3-diiminoisoindoline was used in place of 50 parts of 1,3-diiminoisoindoline, and 40.07 parts of 1,3-dimethylbarbituric acid was used instead of 61.85 parts, and 74.61 parts (a yield of 73%) of Isoindoline Compound 1-12 was obtained.

### (Synthesis Example 1-13)

A reaction operation was performed in the same manner as in Synthesis Example 1-1 except that 46.56 parts of 2-cyano-butyl acetate was used in place of 35.48 parts of 2-cyano-N-methylacetamide of Synthesis Example 1-1, 50 parts of 5-methyl-1,3-diiminoisoindoline was used in place of 50 parts of 1,3-diiminoisoindoline, and 61.47 parts of 1-ethyl-3-methylbarbituric acid was used in place of 61.85 parts of 1,3-dimethylbarbituric acid, and 94.53 parts (a yield of 69%) of Isoindoline Compound 1-13 was obtained.

### (Synthesis Example 1-14)

A reaction operation was performed in the same manner as in Synthesis Example 1-1 except that 20.47 parts of 2-cyano-methyl acetate was used in place of 35.48 parts of 2-cyano-N-methylacetamide of Synthesis Example 1-1, 50 parts of 4-bromo-6-methoxy-1,3-diiminoisoindoline was used in place of 50 parts of 1,3-diiminoisoindoline, and 35.34 parts of 1,3-dimethylbarbituric acid was used instead of 61.85 parts, and 58.77 parts (a yield of 63%) of Isoindoline Compound 1-14 was obtained.

### (Comparative Compound 1)

A reaction operation was performed in the same manner as in Synthesis Example 1-1 except that 50.74 parts of barbituric acid was used in place of 61.85 parts of 1,3-dimethylbarbituric acid of Synthesis Example 1-1, and 102.16 parts (a yield of 88%) of C. I. Pigment Yellow 185 was obtained.

Table 4 shows the structures of isoindoline compounds obtained in Synthesis Examples 1-1 to 1-14 and Comparative Compound 1 together. Here, for example, as shown in the compound of Synthesis Example 1-3, when the relationships between R₁ and R₄, between R₂ and R3, and between R₅ and R₆ were asymmetric, the compound was obtained as a mixture of isomers.

The identification of the obtained isoindoline compounds was performed by comparing the molecular ion peak of the mass spectrum with a mass number (theoretical value) obtained by calculation. The measurement of the molecular ion peak of the mass spectrum was performed using ACQUITY UPLS H-Class (commercially available from Waters) (column used: ACQUITY UPLC BEH C18 Column 130Å, 1.7 µm, 2.1 mm×50 mm)/Ms TAP XEVO TQD. Table 4 shows the theoretical molecular weights of the isoindoline compounds (Synthesis Examples 1-1 to 1-14 and Comparative Compound 1) and the measured values obtained by mass spectrometry. Due to the nature of measurement, for the measured value, since H (proton) of the compound was eliminated, if the value was a mass number of the theoretical molecular weight-(minus) 1, the compound matched.

**[Table 4]**

| | R₁,R₄ | | R₂, R₃ | | R₅, R₆ | | R₇ | X | Theoretical molecular weight | Measured value |
|---|---|---|---|---|---|---|---|---|---|---|
| Synthesis Example 1-1 | H | H | H | H | Me | Me | Me | NH | 365 | 364 |
| Synthesis Example 1-2 | H | H | H | H | Et | Et | Et | NH | 407 | 406 |
| Synthesis Example 1-3 | H | H | t-Bu | H | Me | Et | Me | NH | 435 | 434 |
| Synthesis Example 1-4 | H | H | Cl | H | Bu | Bu | Me | NH | 483 | 482 |
| Synthesis Example 1-5 | H | H | NO₂ | H | Me | Me | Me | NH | 410 | 409 |
| Synthesis Example 1-6 | NO₂ | H | H | H | Me | Me | Me | NH | 410 | 409 |
| Synthesis Example 1-7 | Cl | Cl | Cl | Cl | Cyclohexyl | Cyclohexyl | Me | NH | 637 | 636 |
| Synthesis Example 1-8 | OMe | H | H | H | Me | H | Bu | NH | 423 | 422 |
| Synthesis Example 1-9 | H | H | SMe | H | Me | H | Me | NH | 397 | 396 |
| Synthesis Example 1-10 | OPh | H | H | H | Me | Me | Me | NH | 457 | 456 |
| Synthesis Example 1-11 | H | H | H | H | Me | Me | Me | O | 366 | 365 |
| Synthesis Example 1-12 | H | H | Br | H | Me | Me | Et | O | 458 | 457 |
| Synthesis Example 1-13 | H | H | Me | H | Me | Et | Bu | O | 436 | 435 |
| Synthesis Example 1-14 | OMe | H | H | Br | Me | Me | Me | O | 474 | 473 |
| Comparative Compound 1 | H | H | H | H | H | H | Me | NH | 337 | 336 |

### (Synthesis Example 1-15)

A mixture containing 50.0 parts of Isoindoline Compound 1-1 obtained in Synthesis Example 1-1, 500 parts of sodium chloride, and 85 parts of diethylene glycol was kneaded using a stainless steel 1-gallon kneader (commercially available from Inoue Co., Ltd.) at 60°C for 10 hours to obtain a clay-like kneaded product. The kneaded product was put into 7,000 parts of hot water at 70°C and stirred for 2 hours to obtain a crude slurry. This crude slurry was filtered through a Buechner funnel, and washed by sprinkling 5,000 parts of hot water at 50°C on the filter. Then, the filter was taken out and added to an aqueous solution in which 2 parts of 98% sulfuric acid and 2,000 parts of water were mixed and the mixture was stirred for 1 hour to form a slurry. This slurry was filtered through a Buechner funnel, and washed by sprinkling 5,000 parts of deionized water on the filter. Then, drying and crushing were performed to obtain 45.0 parts of Isoindoline Compound 1-15.

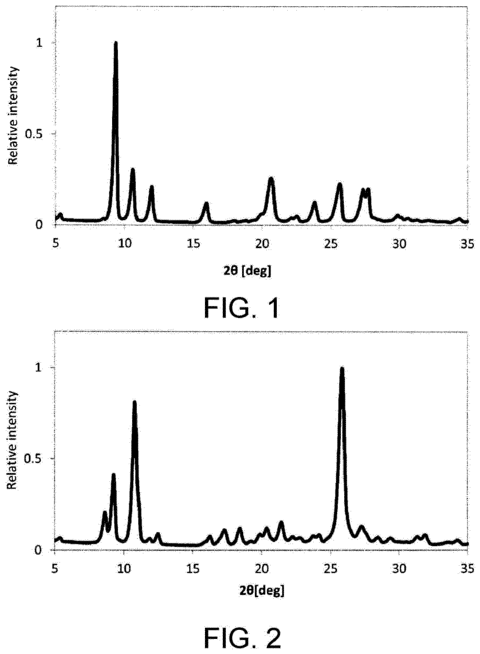

Isoindoline Compound 1-15 obtained as described above had the same structure as Isoindoline Compound 1-1 obtained in Synthesis Example 1-1 (refer to Table 4), but it was a compound obtained by refining Isoindoline Compound 1-1. The X-ray diffraction spectrums of Isoindoline Compounds 1-1 and 1-15 obtained in Synthesis Examples 1-1 and 1-15 with Cu-Ka rays are shown in order in Fig. 1 and Fig. 2. Comparing Fig. 1 and Fig. 2, it can be understood that Isoindoline Compounds 1-1 and 1-15 had different crystal types.

### (Synthesis Example 2-1)

800 parts of water and 800 parts of 80% acetic acid were put into a 3 L 4-neck flask including a reflux cooling tube, a dropping funnel and a stirrer, and the mixture was stirred. 112.96 parts of 1,3-dimethylbarbituric acid was added thereto and the mixture was stirred at 85°C and 1,3-dimethylbarbituric acid was dissolved. 50 parts of 1,3-diiminoisoindoline was added thereto and the mixture was stirred at 85°C. Stirring was performed until the raw material 1,3-diiminoisoindoline disappeared. The disappearance of the raw material was confirmed through UPLC. Then, cooling was performed to room temperature and washing with 2,000 parts of water was then performed three times to obtain a non-volatile content. The non-volatile content was dried in a hot air dryer at 80°C and 139.92 parts (a yield of 96%) of Isoindoline Compound 2-1 was obtained.

### (Synthesis Example 2-2)

1,550 parts of water and 50 parts of 80% acetic acid were put into a 3 L 4-neck flask including a reflux cooling tube, a dropping funnel and a stirrer, and the mixture was stirred. 56.48 parts of 1,3-dimethylbarbituric acid was added thereto and the mixture was stirred at 25°C. 50 parts of 1,3-diiminoisoindoline was added thereto and the mixture was stirred at 25°C. Stirring was performed until the raw material 1,3-diiminoisoindoline disappeared. The disappearance of the raw material was confirmed through UPLC. The reaction slurry was filtered off using a Buechner funnel to obtain a non-volatile content. The non-volatile content was used in the following reaction.

800 parts of water, 800 parts of 80% acetic acid, a total amount of the non-volatile content obtained in the above preparation as a raw material, and 66.62 parts of 1,3-diethyl barbituric acid were put into a 3 L 4-neck flask including a reflux cooling tube, a dropping funnel and a stirrer, and the mixture was stirred at 85°C. Stirring was performed until the non-volatile content used as a raw material disappeared. The disappearance of the raw material was confirmed through UPLC.

Then, cooling was performed to room temperature and washing with 2,000 parts of water was then performed three times to obtain a non-volatile content. The non-volatile content was dried in a hot air dryer at 80°C, and 139.92 parts (a yield of 96%) of Isoindoline Compound 2-2 was obtained.

### (Synthesis Example 2-3)

A reaction operation was performed in the same manner as in Synthesis Example 2-1 except that 81.46 parts of 1,3-dimethylbarbituric acid of Synthesis Example 2-1 was used instead of 112.96 parts, and 50 parts of 5-tert-butyl-1,3-diiminoisoindoline was used in place of 50 parts of 1,3-diiminoisoindoline, and 111.90 parts (a yield of 94%) of Isoindoline Compound 2-3 was obtained.

### (Synthesis Example 2-4)

A reaction operation was performed in the same manner as in Synthesis Example 2-2 except that 38.30 parts of 1,3-dimethylbarbituric acid of Synthesis Example 2-2 was used instead of 56.48 parts, 50 parts of 5,6-dichloro-1,3-diiminoisoindoline was used in place of 50 parts of 1,3-diiminoisoindoline, and 45.18 parts of 1,3-diethyl barbituric acid was used instead of 66.62 parts, and 66.69 parts (a yield of 55%) of Isoindoline Compound 2-4 was obtained.

### (Synthesis Example 2-5)

A reaction operation was performed in the same manner as in Synthesis Example 2-2 except that 51.49 parts of 1,3-dimethylbarbituric acid of Synthesis Example 2-2 was used instead of 56.48 parts, 50 parts of 5-methyl-1,3-diiminoisoindoline was used in place of 50 parts of 1,3-diiminoisoindoline, and 42.24 parts of barbituric acid was used in place of 66.62 parts of 1,3-diethyl barbituric acid, and 65.53 parts (a yield of 51%) of Isoindoline Compound 2-5 was obtained.

### (Synthesis Example 2-6)

A reaction operation was performed in the same manner as in Synthesis Example 2-1 except that 58.9 parts of 1-methyl barbituric acid was used in place of 112.96 parts of 1,3-dimethylbarbituric acid of Synthesis Example 2-1 and 50 parts of 4-thiophenyl-1,3-diiminoisoindoline was used in place of 50 parts of 1,3-diiminoisoindoline, and 90.36 parts (a yield of 91%) of Isoindoline Compound 2-6 was obtained.

### (Synthesis Example 2-7)

A reaction operation was performed in the same manner as in Synthesis Example 2-2 except that 28.33 parts of 1,3-dimethylbarbituric acid of Synthesis Example 2-2 was used instead of 56.48 parts, 50 parts of 4,7-dibutoxy-1,3-diiminoisoindoline was used in place of 50 parts of 1,3-diiminoisoindoline, and 28.33 parts of 1-ethylbarbituric acid was used in place of 66.62 parts of 1,3-diethyl barbituric acid, and 43.12 parts (a yield of 44%) of Isoindoline Compound 2-7 was obtained.

### (Synthesis Example 2-8)

A reaction operation was performed in the same manner as in Synthesis Example 2-1 except that 78.2 parts of 1,3-dimethylbarbituric acid of Synthesis Example 2-1 was used instead of 112.96 parts, and 50 parts of 4-chloro-6-methoxy-1,3-diiminoisoindoline was used in place of 50 parts of 1,3-diiminoisoindoline, and 102.24 parts (a yield of 88%) of Isoindoline Compound 2-8 was obtained.

### (Synthesis Example 2-9)

A reaction operation was performed in the same manner as in Synthesis Example 2-2 except that 43.11 parts of 1,3-dimethylbarbituric acid of Synthesis Example 2-2 was used instead of 56.48 parts, 50 parts of 5-nitro-1,3-diiminoisoindoline was used in place of 50 parts of 1,3-diiminoisoindoline, and 54.73 parts of 1-butyl-3-methylbarbituric acid was used in place of 66.62 parts of 1,3-diethyl barbituric acid, and 48.29 parts (a yield of 36%) of Isoindoline Compound 2-9 was obtained.

### (Comparative Compound 2)

A reaction operation was performed in the same manner as in Synthesis Example 2-1 except that 92.66 parts of barbituric acid was used in place of 112.96 parts of 1,3-dimethylbarbituric acid of Synthesis Example 2-1, and 111.25 parts (a yield of 88%) of C. I. Pigment Yellow 139 was obtained.

Table 5 shows the structures of isoindoline compounds of Synthesis Examples 2-1 to 2-9 and Comparative Compound 2 together. Here, in the isoindoline compounds, for example, as shown in Synthesis Example 2-3, when the relationships between R₁ and R₄, between R₂ and R₃, between R₅ and R₆, and between R₅ and R₉ of the compounds used as raw materials were asymmetric, the final product was obtained as a mixture of isomers.

In the same manner as above, the identification of the obtained isoindoline compounds was performed by comparing the molecular ion peak of the mass spectrum with a mass number (theoretical value) obtained by calculation. Table 5 shows the theoretical molecular weights of the isoindoline compounds (Synthesis Examples 2-1 to 2-9 and Comparative Compound 2) and the measured value obtained by mass spectrometry. Due to the nature of measurement, for the measured value, since H (proton) of the compound was eliminated, if the value was a mass number of the theoretical molecular weight-(minus) 1, the compound matched.

**[Table 5]**

| | R_{1,} R₄ | | R₂, R₃ | | R₅, R₆ | | R₈, R₉ | | Theoretical molecular weight | Measured value |
|---|---|---|---|---|---|---|---|---|---|---|
| Synthesis Example 2-1 | H | H | H | H | Me | Me | Me | Me | 423 | 422 |
| Synthesis Example 2-2 | H | H | H | H | Me | Me | Et | Et | 451 | 450 |
| Synthesis Example 2-3 | H | H | t-Bu | H | Me | Me | Me | Me | 479 | 478 |
| Synthesis Example 2-4 | H | H | Cl | Cl | Me | Me | Et | Et | 519 | 518 |
| Synthesis Example 2-5 | H | H | Me | H | Me | Me | H | H | 409 | 408 |
| Synthesis Example 2-6 | SPh | H | H | H | Me | H | Me | H | 503 | 502 |
| Synthesis Example 2-7 | OBu | OBu | H | H | Et | H | Me | Me | 567 | 566 |
| Synthesis Example 2-8 | OMe | H | H | Cl | Me | Me | Me | Me | 487 | 486 |
| Synthesis Example 2-9 | H | H | NO₂ | H | Me | Bu | Me | Me | 510 | 509 |
| Comparative Compound 2 | H | H | H | H | H | H | H | H | 367 | 366 |

### (Synthesis Example 3-1)

A reaction operation was performed in the same manner as in Synthesis Example 1-1 except that 66.98 parts of 2-(4-oxo-3,4-dihydroquinazoline-2-yl)acetonitrile was used in place of 35.48 parts of 2-cyano-N-methylacetamide of Synthesis Example 1-1, and 118.36 parts (a yield of 76%) of Isoindoline Compound 3-1 was obtained.

### (Synthesis Example 3-2)

A reaction operation was performed in the same manner as in Synthesis Example 1-1 except that 72.05 parts of 2-(3-methyl-4-oxo-3,4-dihydroquinazoline-2-yl)acetonitrile was used in place of 35.48 parts of 2-cyano-N-methylacetamide of Synthesis Example 1-1, and 125.25 parts (a yield of 78%) of Isoindoline Compound 3-2 was obtained.

### (Synthesis Example 3-3)

A reaction operation was performed in the same manner as in Synthesis Example 1-1 except that 73.49 parts of 2-(6-fluoro-4-oxo-3,4-dihydroquinazoline-2-yl)acetonitrile was used in place of 35.48 parts of 2-cyano-N-methylacetamide of Synthesis Example 1-1, and 114.98 parts (a yield of 71%) of Isoindoline Compound 3-3 was obtained.

### (Synthesis Example 3-4)

A reaction operation was performed in the same manner as in Synthesis Example 1-1 except that 66.98 parts of 2-(4-oxo-3,4-dihydroquinazoline-2-yl)acetonitrile was used in place of 35.48 parts of 2-cyano-N-methylacetamide of Synthesis Example 1-1 and 67.41 parts of 1-ethyl-3-methylbarbituric acid was used in place of 61.85 parts of 1,3-dimethylbarbituric acid, and 112.4 parts (a yield of 70%) of Isoindoline Compound 3-4 was obtained.

### (Synthesis Example 3-5)

A reaction operation was performed in the same manner as in Synthesis Example 1-1 except that 51.38 parts of 2-(4-oxo-3,4-dihydroquinazoline-2-yl)acetonitrile was used in place of 35.48 parts of 2-cyano-N-methylacetamide of Synthesis Example 1-1, 50 parts of 5-ethoxy-1,3-diiminoisoindoline was used in place of 50 parts of 1,3-diiminoisoindoline, and 47.45 parts of 1,3-dimethylbarbituric acid was used instead of 61.85 parts, and 90.47 parts (a yield of 69%) of Isoindoline Compound 3-5 was obtained.

### (Synthesis Example 3-6)

A reaction operation was performed in the same manner as in Synthesis Example 1-1 except that 59.18 parts of 2-(3-butyl-4-oxo-3,4-dihydroquinazoline-2-yl)acetonitrile was used in place of 35.48 parts of 2-cyano-N-methylacetamide of Synthesis Example 1-1, 50 parts of 5,6-dichloro-1,3-diiminoisoindoline was used in place of 50 parts of 1,3-diiminoisoindoline, and 41.94 parts of 1,3-dimethylbarbituric acid was used instead of 61.85 parts, and 88.82 parts (a yield of 66%) of Isoindoline Compound 3-6 was obtained.

### (Synthesis Example 3-7)

A reaction operation was performed in the same manner as in Synthesis Example 1-1 except that 47.38 parts of 2-(4-oxo-3,4-dihydroquinazoline-2-yl)acetonitrile was used in place of 35.48 parts of 2-cyano-N-methylacetamide of Synthesis Example 1-1, 50 parts of 4,7-dimethoxy-1,3-diiminoisoindoline was used in place of 50 parts of 1,3-diiminoisoindoline, and 51.61 parts of 1,3-diethyl barbituric acid was used in place of 61.85 parts of 1,3-dimethylbarbituric acid, and 73.71 parts (a yield of 66%) of Isoindoline Compound 3-7 was obtained.

### (Synthesis Example 3-8)

A reaction operation was performed in the same manner as in Synthesis Example 1-1 except that 43.35 parts of 2-(6-chloro-3-methyl-4-oxo-3,4-dihydroquinazoline-2-yl)acetonitrile was used in place of 35.48 parts of 2-cyano-N-methylacetamide of Synthesis Example 1-1, 50 parts of 4,5,6,7-tetrachloro-1,3-diiminoisoindoline was used in place of 50 parts of 1,3-diiminoisoindoline, and 28.88 parts of 1-methyl barbituric acid was used in place of 61.85 parts of 1,3-dimethylbarbituric acid, and 64.84 parts (a yield of 59%) of Isoindoline Compound 3-8 was obtained.

### (Comparative Compound 3)

A reaction operation was performed in the same manner as in Synthesis Example 1-1 except that 66.98 parts of 2-(4-oxo-3,4-dihydroquinazoline-2-yl)acetonitrile was used in place of 35.48 parts of 2-cyano-N-methylacetamide of Synthesis Example 1-1, and 50.74 parts of barbituric acid was used in place of 61.85 parts of 1,3-dimethylbarbituric acid, and 109.54 parts (a yield of 75%) of C. I. Pigment Red 260 was obtained.

Table 6 shows the structures of the isoindoline compounds obtained in Synthesis Examples 3-1 to 3-8 and Comparative Compound 3 together. Here, for example, as shown in Synthesis Example 3-5, when the relationships between R₁ and R₄, between R₂ and R₃, and between R₅ and R₆ of the compounds used as raw materials were asymmetric, the final product was obtained as a mixture of isomers.

In the same manner as above, the identification of the obtained isoindoline compounds was performed by comparing the molecular ion peak of the mass spectrum with a mass number (theoretical value) obtained by calculation. Table 6 shows the theoretical molecular weights of the isoindoline compounds (Synthesis Examples 3-1 to 3-8 and Comparative Compound 3) and the measured values obtained by mass spectrometry. Due to the nature of measurement, for the measured value, since H (proton) of the compound was eliminated, if the value was a mass number of the theoretical molecular weight-(minus) 1, the compound matched.

**[Table 6]**

| | R₁,R₄ | | R₂, R₃ | | R₅, R₆ | | R₁₀ | R₁₁ | R₁₂ | R₁₃ | R₁₄ | Theoretical molecular weight | Measured value |
|---|---|---|---|---|---|---|---|---|---|---|---|---|---|
| Synthesis Example 3-1 | H | H | H | H | Me | Me | H | H | H | H | H | 452 | 451 |
| Synthesis Example 3-2 | H | H | H | H | Me | Me | H | H | H | H | H | 466 | 465 |
| Synthesis Example 3-3 | H | H | H | H | Me | Me | H | H | F | H | H | 470 | 469 |
| Synthesis Example 3-4 | H | H | H | H | Me | Et | H | H | H | H | H | 466 | 465 |
| Synthesis Example 3-5 | H | H | OEt | H | Me | Me | H | H | H | H | H | 496 | 495 |
| Synthesis Example 3-6 | H | H | Cl | Cl | Me | Me | Bu | H | H | H | H | 576 | 575 |
| Synthesis Example 3-7 | OMe | OMe | H | H | Et | Et | H | H | H | H | H | 540 | 539 |
| Synthesis Example 3-8 | Cl | Cl | Cl | Cl | Me | H | Me | H | Cl | H | H | 622 | 621 |
| Comparative Compound 3 | H | H | H | H | H | H | H | H | H | H | H | 424 | 423 |

### <Coloring composition and evaluation of properties thereof>

Coloring compositions for various applications were prepared using the isoindoline compounds synthesized above, and various properties were evaluated.

### < 1 > Coloring composition for plastics and evaluations thereof

The following relates to coloring compositions for plastics prepared using the isoindoline compounds prepared above.

### (Examples A-1 to A-32 and Comparative Examples A-1 to A-3)

First, using the isoindoline compounds synthesized above and resins, as samples used for the test, colored plates in which the coloring power was adjusted to have a concentration of SD1/3 were prepared. Table 7 shows the isoindoline compounds using the examples and the comparative examples. A high density polyethylene (product name: Hizex 2208 J, commercially available from Prime Polymer Co., Ltd.) was used as the resin constituting the test sample. Molding of colored plates was performed at 200°C under a condition in which the retention time in the barrel was as short as possible, and 11 colored plates were obtained. In order to detect the average color difference, the color of each of six 6^{th} to 11^{th} colored plates was measured using a colorimeter that can could a total light flux (CM-700d, commercially available from Konica Minolta, Inc.). The average value of the obtained color measurement value was used as a control (reference value).

Next, after molding conditions were adjusted so that the retention time in the barrel was 5 minutes, 11 colored plates were molded at 300°C. For the obtained colored plates, the color of each of six 6^{th} to 11^{th} plates was measured and the average value of the color measurement values was calculated. A color difference (ΔE ∗) between the reference value and the measured value of the plate molded at 300°C was obtained and evaluated based on the following criteria. The results are shown in Table 7. When the color difference was smaller, the heat resistance was better, and "O" and "Δ" in the following evaluation criteria mean practical levels.

### (Evaluation criteria)

○: ΔE∗ was less than 5.0. Good
△: ΔE∗ was 5.0 or more and less than 10. Practical
× ΔE∗ was 10.0 or more. Not practical

**[Table 7]**

| Example | Isoindoline Compound | ΔE |
|---|---|---|
| Example A-1 | Synthesis Example 1-1 | ○ |
| Example A-2 | Synthesis Example 1-2 | ○ |
| Example A-3 | Synthesis Example 1-3 | ○ |
| Example A-4 | Synthesis Example 1-4 | ○ |
| Example A-5 | Synthesis Example 1-5 | ○ |
| Example A-6 | Synthesis Example 1-6 | ○ |
| Example A-7 | Synthesis Example 1-7 | ○ |
| Example A-8 | Synthesis Example 1-8 | △ |
| Example A-9 | Synthesis Example 1-9 | △ |
| Example A-10 | Synthesis Example 1-10 | ○ |
| Example A-11 | Synthesis Example 1-11 | △ |
| Example A-12 | Synthesis Example 1-12 | ○ |
| Example A-13 | Synthesis Example 1-13 | △ |
| Example A-14 | Synthesis Example 1-14 | ○ |
| Example A-15 | Synthesis Example 1-15 | ○ |
| Comparative Example A-1 | Comparative Compound 1 | × |
| Example A-16 | Synthesis Example 2-1 | ○ |
| Example A-17 | Synthesis Example 2-2 | ○ |
| Example A-18 | Synthesis Example 2-3 | ○ |
| Example A-19 | Synthesis Example 2-4 | ○ |
| Example A-20 | Synthesis Example 2-5 | △ |
| Example A-21 | Synthesis Example 2-6 | △ |
| Example A-22 | Synthesis Example 2-7 | △ |
| Example A-23 | Synthesis Example 2-8 | ○ |
| Example A-24 | Synthesis Example 2-9 | ○ |
| Comparative Example A-2 | Comparative Compound 2 | × |
| Example A-25 | Synthesis Example 3-1 | ○ |
| Example A-26 | Synthesis Example 3-2 | ○ |
| Example A-27 | Synthesis Example 3-3 | ○ |
| Example A-28 | Synthesis Example 3-4 | ○ |
| Example A-29 | Synthesis Example 3-5 | ○ |
| Example A-30 | Synthesis Example 3-6 | ○ |
| Example A-31 | Synthesis Example 3-7 | ○ |
| Example A-32 | Synthesis Example 3-8 | △ |
| Comparative Example A-3 | Comparative Compound 3 | × |

### (Example A-33)

As an example of a coloring composition for plastics, a coloring composition containing Isoindoline Compound (1) and polypropylene as a resin was examined. First, 2 parts of Isoindoline Compound (Synthesis Example 1-1) and 1,000 parts of the polypropylene resin (product name: Prime Polypro J105, commercially available from Prime Polymer Co., Ltd.) were mixed to obtain a coloring composition for plastics. Next, the coloring composition obtained as described above was injection-molded using an injection molding machine of which injection conditions were set to a molding temperature of 220°C and a mold temperature of 40°C to obtain a molded product (plate). As a result of visually observing the molded product, no warpage or deformation was observed and a highly colored yellow plate was obtained.

### (Example A-34)

As an example of a coloring composition for plastics, a coloring composition containing Isoindoline Compound (1) and polyethylene terephthalate as a resin was examined.

First, 2 parts of the isoindoline compound (Synthesis Example 1-1) and 1,000 parts of a polyethylene terephthalate resin (product name: Vylopet EMChaihun307, commercially available from Toyobo Co., Ltd.) were mixed to obtain a coloring composition for plastics. Next, the coloring composition obtained as described above was injection-molded using an injection molding machine of which injection conditions were set to a molding temperature of 275°C and a mold temperature of 85°C to obtain a molded product (plate). As a result of visually observing the molded product, no warpage or deformation was observed and a highly colored yellow plate was obtained.

### <2> Preparation of coloring composition for toner and evaluation thereof

The following relates to a preparation example of a coloring composition for toner (negatively charged toner) that can be used for producing a toner.

### (Example A-35)

2,500 g of the isoindoline compound (Synthesis Example 1-1) and 2,500 g of a thermoplastic polyester resin were mixed and kneaded in a pressurizing kneader. Mixing and kneading were performed at a setting temperature of 120°C for 15 minutes. Then, the obtained kneaded product was taken out from the pressurizing kneader, and additionally kneaded using three rollers with a roller temperature of 95°C. The obtained kneaded product was cooled and then coarsely crushed to a size of 10 mm or less, and thereby a colored resin composition was obtained.

500 g of the colored resin composition obtained as described above, 4,375 g of a thermoplastic polyester resin, 50 g of a 3,5-di-tert-butylsalicylic acid calcium salt compound (charge control agent), and 75 g of an ethylene homopolymer (mold release agent, a molecular weight of 850, Mw/Mn=1.08, a melting point of 107°C) were mixed (at 3,000 rpm for 3 minutes) using a Henschel mixer with a 20 L volume, and the mixture was additionally melted and kneaded using a biaxial kneading extruder at a discharge temperature of 120°C. Then, the kneaded product was cooled and solidified and then coarsely crushed with a hammer mill. Then, the obtained coarsely crushed product was finely crushed using a type I jet mill (IDS-2 type) and then classified to obtain toner base particles.

Then, 2,500 g of the toner base particles obtained above and 12.5 g of hydrophobic titanium oxide (commercially available from Titan Kogyo, Ltd. STT-30A) were mixed with a 10 L Henschel mixer to obtain a negatively charged toner 1.

On the other hand, as a comparison object, a negatively charged toner 2 was obtained in the same manner as in Example A-35 except that Comparative Compound 1 was used in place of the isoindoline compound (Synthesis Example 1-1) of Example A-35.

The obtained negatively charged toner 1 and negatively charged toner 2 were each sliced to a thickness of 0.9 µm using a microtome to form a sample. Then, the pigment dispersion state of each sample was observed using a transmission electron microscope. As a result, it was found that the pigment was more uniformly dispersed and the dispersibility was higher in the negatively charged toner 1 using the compound of Synthesis Example 1-1 than in the negatively charged toner 2 using Comparative Compound 1.

### <3> Solid base paint and evaluation thereof

The following relates to a solid base paint prepared using the isoindoline compounds prepared above and evaluation of weather resistance of the solid base coated plate produced using the same.

### <3-1> Preparation of solid base paint

### (1) Preparation of base paint

### (Example B-1)-Method of preparing base paint 1

First, the following raw materials and 230 parts of steel beads were put into a 225 ml glass bottle and dispersed using a paint shaker (commercially available from Red Devil) for 60 minutes to obtain a mixture.
- Isoindoline Compound (Synthesis Example 1-1): 19 parts
- Acrylic resin (ACRYDIC 47-712, commercially available from DIC): 7.7 parts
- Dispersion solvent (a mixed solvent in which toluene:xylene:butyl acetate: SOLVESSO 150 (commercially available from TonenGeneral Sekiyu K.K.) were mixed at a mass ratio of 3:3:2:2): 40.7 parts

Then, 75.4 parts of ACRYDIC 47-712 and 17.2 parts of a melamine resin (AMIDIR L-1 17-60, commercially available from DIC) were added to the mixture, and the mixture was additionally dispersed for 10 minutes to obtain a dispersion solution.

Then, the steel beads were removed from the dispersion solution to obtain a base paint 1 of the isoindoline compound (Synthesis Example 1-1).

### (Examples B-2 to B-32 and Comparative Examples B-1 to B-3)-Preparation of base paints 2 to 35

Base paints 2 to 35 were obtained in the same manner as in Example B-1 except that Synthesis Examples 1-2 to 1-15, 2-1 to 2-9, and 3-1 to 3-8, and Comparative Compounds 1 to 3 were used in place of the isoindoline compound (Synthesis Example 1-1) in the method of preparing the base paint 1 described in Example B-1.

### (2) Preparation of white paint

The following relates to a preparation example of a white paint used for a solid base paint.

First, the following raw materials and 900 parts of steel beads were put into a 900 ml glass bottle and dispersed using a paint shaker (commercially available from Red Devil) for 60 minutes to obtain a dispersion solution.
- Titanium oxide (titanium oxide TIPAQUE CR90, commercially available from Ishihara Sangyo Kaisha, Ltd.): 66.6 parts
- Acrylic resin (ACRYDIC 47-712, commercially available from DIC): 101.7 parts
- Melamine resin (AMIDIR L-117-60, commercially available from DIC): 21.3 parts
- Dispersion solvent (a mixed solvent in which toluene:xylene:butyl acetate: SOLVESSO 150 (commercially available from TonenGeneral Sekiyu K.K.) were mixed at a mass ratio of 3:3:2:2): 20.9 parts

Then, the steel beads were removed from the dispersion solution to obtain a white paint.

### (3) Preparation of solid base paint

### (Example C-1)-Preparation of solid base paint 1

The following components were stirred using a high-speed stirrer to obtain a solid base paint 1.
- Base paint 1 prepared in Example B-1: 10 parts
- White paint: 31.9 parts

### (Examples C-2 to C-32 and Comparative Examples C-1 to C-3)-Preparation of solid base paints 2 to 35

Solid base paints 2 to 35 were obtained in the same manner as in Example C-1 except that base paints 2 to 35 were used in place of the base paint 1 of Example C-1.

Here, the isoindoline compounds of the base paints used in the solid base paints prepared in the examples and comparative examples are as shown in Table 8.

**[Table 8]**

| Example | Solid paint | Base paint | Isoindoline compound |
|---|---|---|---|
| Example C-1 | Solid base paint 1 | Base paint 1 | Synthesis Example 1-1 |
| Example C-2 | Solid base paint 2 | Base paint 2 | Synthesis Example 1-2 |
| Example C-3 | Solid base paint 3 | Base paint 3 | Synthesis Example 1-3 |
| Example C-4 | Solid base paint 4 | Base paint 4 | Synthesis Example 1-4 |
| Example C-5 | Solid base paint 5 | Base paint 5 | Synthesis Example 1-5 |
| Example C-6 | Solid base paint 6 | Base paint 6 | Synthesis Example 1-6 |
| Example C-7 | Solid base paint 7 | Base paint 7 | Synthesis Example 1-7 |
| Example C-8 | Solid base paint 8 | Base paint 8 | Synthesis Example 1-8 |
| Example C-9 | Solid base paint 9 | Base paint 9 | Synthesis Example 1-9 |
| Example C-10 | Solid base paint 10 | Base paint 10 | Synthesis Example 1-10 |
| Example C-11 | Solid base paint 11 | Base paint 11 | Synthesis Example 1-11 |
| Example C-12 | Solid base paint 12 | Base paint 12 | Synthesis Example 1-12 |
| Example C-13 | Solid base paint 13 | Base paint 13 | Synthesis Example 1-13 |
| Example C-14 | Solid base paint 14 | Base paint 14 | Synthesis Example 1-14 |
| Example C-15 | Solid base paint 15 | Base paint 15 | Synthesis Example 1-15 |
| Comparative Example C-1 | Solid base paint 16 | Base paint 16 | Comparative Compound 1 |
| Example C-16 | Solid base paint 17 | Base paint 17 | Synthesis Example 2-1 |
| Example C-17 | Solid base paint 18 | Base paint 18 | Synthesis Example 2-2 |
| Example C-18 | Solid base paint 19 | Base paint 19 | Synthesis Example 2-3 |
| Example C-19 | Solid base paint 20 | Base paint 20 | Synthesis Example 2-4 |
| Example C-20 | Solid base paint 21 | Base paint 21 | Synthesis Example 2-5 |
| Example C-21 | Solid base paint 22 | Base paint 22 | Synthesis Example 2-6 |
| Example C-22 | Solid base paint 23 | Base paint 23 | Synthesis Example 2-7 |
| Example C-23 | Solid base paint 24 | Base paint 24 | Synthesis Example 2-8 |
| Example C-24 | Solid base paint 25 | Base paint 25 | Synthesis Example 2-9 |
| Comparative Example C-2 | Solid base paint 26 | Base paint 26 | Comparative Example 2 |
| Example C-25 | Solid base paint 27 | Base paint 27 | Synthesis Example 3-1 |
| Example C-26 | Solid base paint 28 | Base paint 28 | Synthesis Example 3-2 |
| Example C-27 | Solid base paint 29 | Base paint 29 | Synthesis Example 3-3 |
| Example C-28 | Solid base paint 30 | Base paint 30 | Synthesis Example 3-4 |
| Example C-29 | Solid base paint 31 | Base paint 31 | Synthesis Example 3-5 |
| Example C-30 | Solid base paint 32 | Base paint 32 | Synthesis Example 3-6 |
| Example C-31 | Solid base paint 33 | Base paint 33 | Synthesis Example 3-7 |
| Example C-32 | Solid base paint 34 | Base paint 34 | Synthesis Example 3-8 |
| Comparative Example C-3 | Solid base paint 35 | Base paint 35 | Comparative Compound 3 |

### (4) Preparation of top coat clear paint

The following raw materials were stirred using a high-speed stirrer to obtain a top coat clear paint.
- Acrylic resin (ACRYDIC 44-179, commercially available from DIC): 120 parts
- Melamine resin (AMIDIR L117-60, commercially available from DIC): 30 parts
- Diluting solvent (a mixed solvent in which toluene, xylene, SOLVESSO 150 (commercially available from TonenGeneral Sekiyu K.K.), ethyl 3-ethoxypropionate, and ethyl acetate were mixed at a mass ratio of 3:2:2:1:2): 50 parts

### <3-2> Production of solid base coated plate and evaluation of weather resistance

The following examples and comparative examples relate to production of solid base coated plates using the solid base paint and top coat clear paint prepared above and evaluation of the coated plate.

### (Example D-1)-Production of solid base coated plate 1

The solid base paint 1 was sprayed with a spray gun and coated on the steel plate. In order to adjust the viscosity so that the paint was easily sprayed, a diluting solvent (a mixed solvent in which toluene, xylene, SOLVESSO 150 (commercially available from TonenGeneral Sekiyu K.K.), ethyl 3-ethoxypropionate, and ethyl acetate were mixed at a mass ratio of 3:2:2:1:2) of which the mass was the same as that of the solid base paint was appropriately mixed as a guide with the solid base paint.

Coating was performed 9 times, and the top coat clear paint was then sprayed 6 times. Then, drying was performed at 25°C for 8 hours and drying was then performed at 140°C for 30 minutes to obtain a solid base coated plate 1.

### (Examples D-2 to D-32 and Comparative Examples D-1 to D-3)-Production of solid base coated plates 2 to 35

Solid base coated plates 2 to 35 were obtained in the same manner as in Example D-1 except that the solid base paints 2 to 35 were used in place of the solid base paint 1 of Example D-1.

Using the solid base coated plates 1 to 35 produced in the examples and the comparative examples, a weather resistance test was performed as follows.

### (Evaluation method of weather resistance test)

The weather resistance test was performed using a super accelerated weather resistance tester (EYE Super Xenon Tester SUV-W151, commercially available from Iwasaki Electric Co., Ltd.) under conditions of 90 mW/cm², 48 hours (2 cycles of 12 hours day and night) and 96 hours (4 cycles of 12 hours day and night). The coated plate before and after the weather resistance test was visually observed, and the weather resistance was evaluated based on the following criteria. The results are shown in Table 9. It was thought that, when there was less change in color, the weather resistance was better, and"O" and "Δ" in the following evaluation criteria mean practical levels.

### (Evaluation criteria)

O: There was no change in color
△: The color faded to white
×: The color turned black

**[Table 9]**

| Example | Solid coated plate | 48 hours | 96 hours |
|---|---|---|---|
| Example D-1 | Solid base coated plate 1 | O | △ |
| Example D-2 | Solid base coated plate 2 | O | △ |
| Example D-3 | Solid base coated plate 3 | O | △ |
| Example D-4 | Solid base coated plate 4 | O | △ |
| Example D-5 | Solid base coated plate 5 | O | O |
| Example D-6 | Solid base coated plate 6 | O | O |
| Example D-7 | Solid base coated plate 7 | △ | △ |
| Example D-8 | Solid base coated plate 8 | △ | △ |
| Example D-9 | Solid base coated plate 9 | △ | △ |
| Example D-10 | Solid base coated plate 10 | O | △ |
| Example D-11 | Solid base coated plate 11 | △ | △ |
| Example D-12 | Solid base coated plate 12 | O | △ |
| Example D-13 | Solid base coated plate 13 | △ | △ |
| Example D-14 | Solid base coated plate 14 | O | △ |
| Example D-15 | Solid base coated plate 15 | △ | △ |
| Comparative Example D-1 | Solid base coated plate 16 | × | × |
| Example D-16 | Solid base coated plate 17 | O | △ |
| Example D-17 | Solid base coated plate 18 | O | △ |
| Example D-18 | Solid base coated plate 19 | O | △ |
| Example D-19 | Solid base coated plate 20 | O | △ |
| Example D-20 | Solid base coated plate 21 | △ | △ |
| Example D-21 | Solid base coated plate 22 | △ | △ |
| Example D-22 | Solid base coated plate 23 | △ | △ |
| Example D-23 | Solid base coated plate 24 | O | O |
| Example D-24 | Solid base coated plate 25 | O | △ |
| Comparative Example D-2 | Solid base coated plate 26 | × | × |
| Example D-25 | Solid base coated plate 27 | O | △ |
| Example D-26 | Solid base coated plate 28 | O | △ |
| Example D-27 | Solid base coated plate 29 | O | △ |
| Example D-28 | Solid base coated plate 30 | O | △ |
| Example D-29 | Solid base coated plate 31 | O | △ |
| Example D-30 | Solid base coated plate 32 | O | △ |
| Example D-31 | Solid base coated plate 33 | O | △ |
| Example D-32 | Solid base coated plate 34 | O | △ |
| Comparative Example D-3 | Solid base coated plate 35 | × | × |

### <4> Aqueous pigment dispersion and evaluation of dispersion stability thereof

The following examples and comparative examples relate to an aqueous pigment dispersion (hereinafter referred to as an aqueous dispersion) prepared using the isoindoline compounds prepared above and evaluation of dispersion stability thereof.

### <4-1> Preparation of aqueous dispersion

### (Example E-1)-Preparation of aqueous dispersion 1

The following raw materials and 70 parts of zirconia beads with a diameter of 1.25 mm were put into a 70 ml glass bottle and dispersed using a paint shaker (commercially available from Red Devil) for 60 minutes to obtain a dispersion solution.
- Isoindoline Compound (Synthesis Example 1-1): 3.15 parts
- Polyester-modified acrylic acid polymer (ADDITOL XW 6528, commercially available from Allnex): 5.25 parts
- Moistening agent (ADDITOL XW 6374, commercially available from Allnex): 0.95 parts
- Antifoaming agent (ADDITOL XW 6211, commercially available from Allnex): 0.63 parts
- Deionized water: 21.52 parts

Then, the zirconia beads were removed from the dispersion solution to obtain an aqueous dispersion 1.

### (Examples E-2 to E-32 and Comparative Examples E-1 to E-3)-Preparation of aqueous dispersions 2 to 35

As shown in Table 10, aqueous dispersions 2 to 35 were obtained in the same manner as in Example E-1 except that Synthesis Examples 1-2 to 1-15, Comparative Compound 1, Synthesis Examples 2-1 to 2-9, Comparative Compound 2, and Synthesis Examples 3-1 to 3-8, and the compound of Comparative Compound 3 were sequentially used in place of the isoindoline pigment (Synthesis Example 1-1) of Example E-1.

### <4-2> Evaluation of dispersion stability

The dispersion stability of the aqueous dispersions obtained in Examples E-1 to E-32, Comparative Examples E-1 to E-3 was evaluated according to the following method.

### (Method of evaluating initial viscosity and viscosity stability)

The initial viscosity of the obtained aqueous dispersion at 25°C was measured using an E type viscometer ("ELD type viscometer," commercially available from Toki Sangyo Co., Ltd.). Similarly, the viscosities after 1 week had elapsed at 25°C and after acceleration was performed for 1 week at 50°C were measured. Based on the obtained measured value, the viscosity increase rate with respect to the initial viscosity was calculated, and used as one index of viscosity stability, and evaluated based on the following evaluation criteria. The results are shown in Table 10. When the initial viscosity was lower, the dispersibility was better. In addition, when the viscosity increase rate was smaller, the dispersion stability was better. "O" and "△" in the following evaluation criteria mean practical levels.

### (Evaluation criteria of initial viscosity)

O: The initial viscosity was less than 6.0 mPa·s.
△: The initial viscosity was 6.0 mPa·s or more and less than 10.0.
×: The initial viscosity was 10.0 mPa·s or more.

### (Evaluation criteria of viscosity stability)

O: The viscosity increase rate was less than 20%.
△: The viscosity increase rate was 20% or more and less than 40%.
×: The viscosity increase rate was 40% or more.

**[Table 10]**

| Example | Isoindoline Compound | Initial viscosity | 25°C for 1 week | 50°C for 1 week |
|---|---|---|---|---|
| Example E-1 | Synthesis Example 1-1 | O | O | O |
| Example E-2 | Synthesis Example 1-2 | O | O | O |
| Example E-3 | Synthesis Example 1-3 | O | O | △ |
| Example E-4 | Synthesis Example 1-4 | O | O | △ |
| Example E-5 | Synthesis Example 1-5 | O | O | O |
| Example E-6 | Synthesis Example 1-6 | O | O | O |
| Example E-7 | Synthesis Example 1-7 | △ | O | △ |
| Example E-8 | Synthesis Example 1-8 | △ | △ | △ |
| Example E-9 | Synthesis Example 1-9 | △ | △ | △ |
| Example E-10 | Synthesis Example 1-10 | O | O | △ |
| Example E-11 | Synthesis Example 1-11 | O | O | △ |
| Example E-12 | Synthesis Example 1-12 | O | O | △ |
| Example E-13 | Synthesis Example 1-13 | O | O | △ |
| Example E-14 | Synthesis Example 1-14 | O | O | △ |
| Example E-15 | Synthesis Example 1-15 | O | O | O |
| Comparative Example E-1 | Comparative Compound 1 | × | × | × |
| Example E-16 | Synthesis Example 2-1 | O | O | O |
| Example E-17 | Synthesis Example 2-2 | O | O | △ |
| Example E-18 | Synthesis Example 2-3 | O | O | O |
| Example E-19 | Synthesis Example 2-4 | O | O | O |
| Example E-20 | Synthesis Example 2-5 | O | O | △ |
| Example E-21 | Synthesis Example 2-6 | O | O | △ |
| Example E-22 | Synthesis Example 2-7 | O | O | △ |
| Example E-23 | Synthesis Example 2-8 | O | O | O |
| Example E-24 | Synthesis Example 2-9 | O | △ | △ |
| Comparative Example E-2 | Comparative Compound 2 | △ | × | × |
| Example E-25 | Synthesis Example 3-1 | O | O | O |
| Example E-26 | Synthesis Example 3-2 | O | O | O |
| Example E-27 | Synthesis Example 3-3 | O | O | O |
| Example E-28 | Synthesis Example 3-4 | O | O | △ |
| Example E-29 | Synthesis Example 3-5 | O | O | O |
| Example E-30 | Synthesis Example 3-6 | O | O | △ |
| Example E-31 | Synthesis Example 3-7 | O | O | △ |
| Example E-32 | Synthesis Example 3-8 | O | O | △ |
| Comparative Example E-3 | Comparative Compound 3 | × | × | × |

### <5> Evaluation of aqueous paints and their coatings

The following relates to evaluation of hue stability of the aqueous paints using the aqueous dispersions prepared in the examples and the comparative examples above and PET films produced using the aqueous paints. Here, the aqueous dispersions obtained in Examples E-1 to E-32 above are shown as aqueous dispersions E-1 to E-32, and the aqueous dispersions obtained in Comparative Examples E-1 to E-3 are shown as comparative aqueous dispersions E-1 to E-3.

### <5-1> Preparation of aqueous paint

### (Example F-1)

### (1) Preparation of aqueous paint 1-1

The following raw materials were stirred using a high-speed stirrer to obtain an aqueous paint 1-1 (stored at 25°C for 1 week).
- Aqueous dispersion E-1 (stored at 25°C for 1 week): 1.4 parts
- Acrylic resin (an acid value of 65.0, an OH value of 50, Mw=15,000, a non-volatile content of 35%): 13.6 parts
- Melamine resin (CYMEL 325, commercially available from Allnex): 3.4 parts

### (2) Preparation of aqueous paint 1-2

The following raw materials were stirred using a high-speed stirrer to obtain an aqueous paint 1-2 (stored at 50°C for 1 week).
- Aqueous dispersion E-1 (stored at 50°C for 1 week): 1.4 parts
- Acrylic resin (an acid value of 65.0, an OH value 50, Mw=15,000, a non-volatile content of 35%): 13.6 parts
- Melamine resin (CYMEL 325, commercially available from Allnex): 3.4 parts

### (Examples F-2 to F-32 and Comparative Examples F-1 to F-3)-Preparation of aqueous paints 2 to 35

Aqueous paints 2-1 to 35-1 were obtained in the same manner as in Example F-1 except that the aqueous dispersions E-2 to 15, the comparative aqueous dispersion E-1, the aqueous dispersions E-16 to 24, the comparative aqueous dispersion E-2, the aqueous dispersions E-25 to 32, and the comparative aqueous dispersion E-3 (each stored at 25°C for 1 week) were sequentially used in place of the aqueous dispersion E-1 (stored at 25°C for 1 week) of Example F-1.

In addition, aqueous paints 2-2 to 35-2 were obtained in the same manner as in Example F-1 except that the aqueous dispersions E-2 to 15, the comparative aqueous dispersion E-1, the aqueous dispersions E-16 to 24, the comparative aqueous dispersion E-2, the aqueous dispersions E-25 to 32, and the comparative aqueous dispersion E-3 (each stored at 50°C for 1 week) were sequentially used in place of the aqueous dispersion E-1 (stored at 50°C for 1 week) of Example F-1.

### <5-2> Production of PET film coating

### (Example G-1)-Production of PET film coating 1

The aqueous paint 1-1 and the aqueous paint 1-2 were applied to the PET film using a 0.007INCH applicator. After the application, the PET film was dried at room temperature for 18 hours. Then, drying was performed at 60°C for 5 minutes and at 140°C for 20 minutes to obtain a PET film coating 1.

### (Examples G-2 to G-32 and Comparative Examples G-1 to G-3)-Production of PET film coating 2 to 35

PET film coatings 2 to 35 were obtained in the same manner as in Example G-1 except that aqueous paints 2-1 to 35-1, and 2-2 to 35-2 were used in place of the aqueous paint 1-1 and the aqueous paint 1-2 of Example G-1.

### <5-3> Evaluation of PET film coating

The hue stability of the PET film coatings obtained in Examples G-1 to G-32 and Comparative Examples G-1 to G-3 was evaluated according to the following method. Thereby, it was possible to evaluate the dispersibility.

### (Method of evaluating hue stability)

Using a colorimeter (CM-700d, commercially available from Konica Minolta, Inc.), the color of the PET films coated with a paint of an aqueous dispersion stored at 25°C for 1 week and with a paint of an aqueous dispersion stored a 50°C for 1 week was measured, and the color difference (ΔE∗) therebetween was obtained and determined based on the following criteria. The results are shown in Table 11. It was thought that, when the color difference was smaller, the coloring material had better dispersion stability, and "O" and "△" in the following evaluation criteria mean practical levels.

### (Evaluation criteria)

O: ΔE∗ was less than 1.5.
△: ΔE∗ was 1.5 or more and less than 3.0.
× ΔE∗ was 3.0 or more

**[Table 11]**

| Example | Isoindoline Compound | ΔE∗ |
|---|---|---|
| Example G-1 | Synthesis Example 1-1 | O |
| Example G-2 | Synthesis Example 1-2 | O |
| Example G-3 | Synthesis Example 1-3 | △ |
| Example G-4 | Synthesis Example 1-4 | △ |
| Example G-5 | Synthesis Example 1-5 | O |
| Example G-6 | Synthesis Example 1-6 | O |
| Example G-7 | Synthesis Example 1-7 | △ |
| Example G-8 | Synthesis Example 1-8 | △ |
| Example G-9 | Synthesis Example 1-9 | △ |
| Example G-10 | Synthesis Example 1-10 | △ |
| Example G-11 | Synthesis Example 1-11 | △ |
| Example G-12 | Synthesis Example 1-12 | △ |
| Example G-13 | Synthesis Example 1-13 | △ |
| Example G-14 | Synthesis Example 1-14 | △ |
| Example G-15 | Synthesis Example 1-15 | O |
| Comparative Example G-1 | Comparative Compound 1 | × |
| Example G-16 | Synthesis Example 2-1 | O |
| Example G-17 | Synthesis Example 2-2 | △ |
| Example G-18 | Synthesis Example 2-3 | O |
| Example G-19 | Synthesis Example 2-4 | O |
| Example G-20 | Synthesis Example 2-5 | △ |
| Example G-21 | Synthesis Example 2-6 | △ |
| Example G-22 | Synthesis Example 2-7 | △ |
| Example G-23 | Synthesis Example 2-8 | O |
| Example G-24 | Synthesis Example 2-9 | △ |
| Comparative Example G-2 | Comparative Compound 2 | × |
| Example G-25 | Synthesis Example 3-1 | O |
| Example G-26 | Synthesis Example 3-2 | O |
| Example G-27 | Synthesis Example 3-3 | O |
| Example G-28 | Synthesis Example 3-4 | △ |
| Example G-29 | Synthesis Example 3-5 | O |
| Example G-30 | Synthesis Example 3-6 | △ |
| Example G-31 | Synthesis Example 3-7 | △ |
| Example G-32 | Synthesis Example 3-8 | △ |
| Comparative Example G-3 | Comparative Compound 3 | × |

### <6> Aqueous inkjet ink and evaluation thereof

The following relates to aqueous inkjet ink dispersions prepared using the isoindoline compound prepared above, aqueous inkjet inks using the same, and evaluation thereof.

### <6-1> Preparation of aqueous inkjet ink dispersion

### (Example H-1)-Preparation of aqueous IJ dispersion 1

- Isoindoline Compound (Synthesis Example 1-1): 19.0 parts
- Styrene-acrylic acid copolymer (Joncryl 61J, commercially available from BASF Japan Ltd.): 16.4 parts
- Surfactant (Emulgen 420, commercially available from Kao Corporation,): 5.0 parts
- Deionized water: 59.6 parts

200 parts of zirconia beads with a diameter of 1.25 mm were put into a 200 ml glass bottle and dispersed using a paint shaker (commercially available from Red Devil) for 6 hours. Then, the zirconia beads were removed from the dispersion solution to obtain an aqueous inkjet ink dispersion 1 (aqueous IJ dispersion 1).

### (Examples H-2 to H-32 and Comparative Examples H-1 to H-3)-Preparation of aqueous IJ dispersions 2 to 35

Aqueous inkjet ink dispersions 2 to 35(aqueous IJ dispersions 2 to 35) were obtained in the same manner as in Example H-1 except that, as shown in Table 12, Synthesis Examples 1-2 to 1-15, 2-1 to 2-9, 3-1 to 3-8, and Comparative Compounds 1 to 3 were used in place of the Isoindoline Compound (Synthesis Example 1-1) of Example H-1.

### <6-2> Preparation of aqueous inkjet ink

The following relates to preparation of aqueous inkjet inks using the aqueous IJ dispersions 1 to 35 prepared above.

### (Example I-1)-Preparation of aqueous inkjet ink 1

The following raw materials were stirred and mixed using a high-speed mixer for 30 minutes to obtain a mixture.
- Aqueous IJ dispersion1 (Example H-1): 12.5 parts
- Styrene-acrylic acid copolymer (Emapoly TYN-40, commercially available from Gifu Shellac Manufacturing Co., Ltd., a non-volatile content of 44.8%): 2.5 parts
- Surfactant (Emulgen 420, commercially available from Kao Corporation): 2.0 parts
- Deionized water: 64.9 parts

Then, diethylene glycol monobutyl ether was appropriately added to the mixture, the viscosity at 25°C was adjusted to 2.5 mPa·s (25°C), the surface tension was adjusted to 40 mN/m, filtering was then performed using a 1.0 µm membrane filter, and filtering was additionally performed using a 0.45 µm membrane filter to obtain an aqueous ink jet ink 1.

### (Examples 1-2 to 1-32 and Comparative Examples 1-1 to I-3)-Preparation of aqueous inkjet inks 2 to 35

Aqueous inkjet inks 2 to 35 were obtained in the same manner as in Example 1-1 except that the aqueous IJ dispersions 2 to 35 were used in place of the aqueous IJ dispersion1 of Example I-1.

### <6-3> Evaluation of aqueous inkjet ink

The viscosity stability of the aqueous inkjet inks 1 to 35 prepared in the examples and the comparative examples was evaluated as follows.

### (Method of evaluating viscosity stability)

The initial viscosity of each aqueous inkjet ink at 25°C was measured using an E type viscometer ("ELD type viscometer," commercially available from Toki Sangyo Co., Ltd.). Similarly, the viscosities after 4 weeks had elapsed at 25°C and after acceleration was performed for 4 weeks at 50°C were measured. Using each measured value, the viscosity increase rate with respect to the initial viscosity was calculated, and used as one index of viscosity stability, and evaluated based on the following criteria. The results are shown in Table 12. It was thought that, when the viscosity increase rate was smaller, the viscosity stability was better, and"O" and "△" in the following evaluation criteria mean practical levels.

### (Evaluation criteria of viscosity stability)

O: The viscosity increase rate was less than 20%.
△: The viscosity increase rate was 20% or more and less than 40%.
×: The viscosity increase rate was 40% or more.

**[Table 12] (continued)**

| Example | Aqueous IJ dispersion | Isoindoline Compound | 25°C for 4 weeks | 50°C for 4 weeks |
|---|---|---|---|---|
| Example I-1 | Aqueous IJ dispersion 1 | Synthesis Example 1-1 | O | O |
| Example 1-2 | Aqueous IJ dispersion 2 | Synthesis Example 1-2 | O | O |
| Example 1-3 | Aqueous IJ dispersion 3 | Synthesis Example 1-3 | O | △ |
| Example 1-4 | Aqueous IJ dispersion 4 | Synthesis Example 1-4 | O | △ |
| Example 1-5 | Aqueous IJ dispersion 5 | Synthesis Example 1-5 | O | O |
| Example 1-6 | Aqueous IJ dispersion 6 | Synthesis Example 1-6 | O | O |
| Example 1-7 | Aqueous IJ dispersion 7 | Synthesis Example 1-7 | O | △ |
| Example 1-8 | Aqueous IJ dispersion 8 | Synthesis Example 1-8 | △ | △ |
| Example 1-9 | Aqueous IJ dispersion 9 | Synthesis Example 1-9 | △ | △ |
| Example I-10 | Aqueous IJ dispersion 10 | Synthesis Example 1-10 | O | △ |
| Example I-11 | Aqueous IJ dispersion 11 | Synthesis Example 1-11 | O | △ |
| Example I-12 | Aqueous IJ dispersion 12 | Synthesis Example 1-12 | O | △ |
| Example I-13 | Aqueous IJ dispersion 13 | Synthesis Example 1-13 | O | △ |
| Example I-14 | Aqueous IJ dispersion 14 | Synthesis Example 1-14 | O | △ |
| Example I-15 | Aqueous IJ dispersion 15 | Synthesis Example 1-15 | O | ○ |
| Comparative Example I-1 | Aqueous IJ dispersion 16 | Comparative Compound 1 | × | × |
| Example I-16 | Aqueous IJ dispersion 17 | Synthesis Example 2-1 | O | O |
| Example I-17 | Aqueous IJ dispersion 18 | Synthesis Example 2-2 | O | △ |
| Example I-18 | Aqueous IJ dispersion 19 | Synthesis Example 2-3 | O | O |
| Example I-19 | Aqueous IJ dispersion 20 | Synthesis Example 2-4 | O | O |
| Example I-20 | Aqueous IJ dispersion 21 | Synthesis Example 2-5 | O | △ |
| Example I-21 | Aqueous IJ dispersion 22 | Synthesis Example 2-6 | O | △ |
| Example I-22 | Aqueous IJ dispersion 23 | Synthesis Example 2-7 | O | △ |
| Example I-23 | Aqueous IJ dispersion 24 | Synthesis Example 2-8 | O | O |
| Example I-24 | Aqueous IJ dispersion 25 | Synthesis Example 2-9 | △ | △ |
| Comparative Example I-2 | Aqueous IJ dispersion 26 | Comparative Compound 2 | × | × |
| Example I-25 | Aqueous IJ dispersion 27 | Synthesis Example 3-1 | O | O |
| Example I-26 | Aqueous IJ dispersion 28 | Synthesis Example 3-2 | O | O |
| Example I-27 | Aqueous IJ dispersion 29 | Synthesis Example 3-3 | O | O |
| Example I-28 | Aqueous IJ dispersion 30 | Synthesis Example 3-4 | O | △ |
| Example I-29 | Aqueous IJ dispersion 31 | Synthesis Example 3-5 | O | O |
| Example I-30 | Aqueous IJ dispersion 32 | Synthesis Example 3-6 | O | △ |
| Example I-31 | Aqueous IJ dispersion 33 | Synthesis Example 3-7 | O | △ |
| Example I-32 | Aqueous IJ dispersion 34 | Synthesis Example 3-8 | O | △ |

In addition, the aqueous inkjet inks 1 to 35 prepared in the examples and the comparative examples were filled in a cartridge of a printer HG5130 (commercially available from Seiko Epson Corporation), and a printing test was performed. When the aqueous inkjet inks of Comparative Examples 1-1 to 1-3 were used, nozzle clogging occurred and printing was not possible. On the other hand, when the aqueous inkjet inks of Examples 1-1 to 1-32 were used, there was no nozzle clogging and neat printing was possible.

Based on the above results, it can be understood that, according to the present invention, when Isoindoline Compound (1) is used, it is possible to provide a coloring composition which has various excellent properties and can be suitably used for various applications. For example, in the form of colorants for plastics and plastic molding materials, it is possible to provide a colorant having excellent heat resistance and it is possible to efficiently provide a plastic molded product because the heat resistance is high. In addition, in the form of toners, it is possible to provide a toner having excellent dispersibility of pigments. In addition, in the form of paints, it is possible to provide a coating having excellent weather resistance, and discoloration can be inhibited in all cases. In addition, in the form of aqueous pigment dispersions, it is possible to improve the initial viscosity and the storage stability. In addition, in the form of aqueous inkjet inks, it is possible to provide an ink which has good storage stability, is less likely to cause nozzle clogging, and can perform high-quality printing.

## Claims

1. A coloring composition including a pigment containing an isoindoline compound represented by following Formula (1) and a dispersion medium: 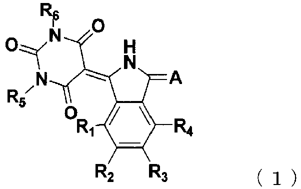 [in the formula, R₁ to R₄ each independently represent a hydrogen atom, a halogen atom, a nitro group, an alkyl group, an alkoxy group, an aryl group, an aryloxy group, a thioalkyl group, or a thioaryl group,
R₅ and R₆ each independently represent a hydrogen atom or an alkyl group, and at least one of R₅ and R₆ represents an alkyl group,
A represent a group represented by following Formula (2), following Formula (3), or following Formula (4), 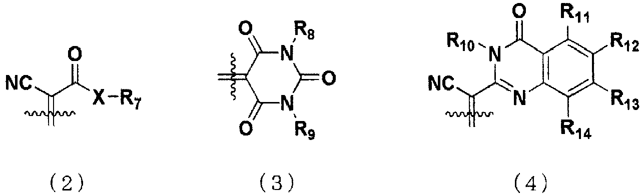
in the formulae, X represents -O- or -NH-, and R₇ represents an alkyl group or an aryl group,
R₅ to R₁₄ each independently represent a hydrogen atom, a halogen atom, an alkyl group, an alkoxy group, or an aryl group].

2. The coloring composition according to claim 1, wherein the dispersion medium contains a resin.

3. A colorant for plastics including the coloring composition according to claim 1 or 2.

4. A plastic molded product including the colorant for plastics according to claim 3.

5. A toner including the coloring composition according to claim 1 or 2.

6. The coloring composition according to claim 1,
wherein the dispersion medium contains a resin and a solvent.

7. The coloring composition according to claim 6,
wherein the solvent contains water.

8. A paint including the coloring composition according to claim 6 or 7.

9. A printing ink including the coloring composition according to claim 6 or 7.

10. An inkjet ink including the coloring composition according to claim 6 or 7.
